(19) Europäisches Patentamt

European Patent Office

Office européen des brevets

(11) **EP 0 941 315 B1**

(12) **EUROPEAN PATENT SPECIFICATION**

(45) Date of publication and mention
of the grant of the patent:
**01.03.2006 Bulletin 2006/09**

(51) Int Cl.:
*C12N 15/10* (2006.01)    *C07K 14/35* (2006.01)
*C07K 19/00* (2006.01)    *C12P 21/00* (2006.01)
*A61K 47/48* (2006.01)    *A61K 39/35* (2006.01)
*A61K 39/145* (2006.01)    *A61K 39/35* (2006.01)
*A61K 39/04* (2006.01)    *A61K 39/145* (2006.01)
*A61K 39/04* (2006.01)

(21) Application number: **97945684.5**

(22) Date of filing: **25.11.1997**

(86) International application number:
**PCT/CA1997/000897**

(87) International publication number:
**WO 1998/023735 (04.06.1998 Gazette 1998/22)**

(54) **FUSION PROTEINS CONTAINING STRESS PROTEINS FOR INDUCING IMMUNE RESPONSES**

FUSIONSPROTEINE, DIE STRESSPROTEINE BEINHALTEN, ZUM HERVORRUFEN EINER IMMUNANTWORT

PROTEINES DE FUSION CONTENANT DES PROTEINES DE STRESS POUR INDUIRE UNE REPONSE IMMUNITAIRE

(84) Designated Contracting States:
**AT BE CH DE DK ES FI FR GB GR IE IT LI LU MC NL PT SE**

(30) Priority: **26.11.1996 US 756621**

(43) Date of publication of application:
**15.09.1999 Bulletin 1999/37**

(73) Proprietor: **Stressgen Biotechnologies Corporation
Victoria,
British Columbia V8Z 4B9 (CA)**

(72) Inventors:
• **MIZZEN, Lee
Victoria, British Columbia V8S 2C4 (CA)**
• **ANTHONY, Lawrence, S., D.
Victoria, British Columbia V8T 4E1 (CA)**

(74) Representative: **Vossius & Partner
Postfach 86 07 67
81634 München (DE)**

(56) References cited:
WO-A-94/03208    WO-A-94/29459
WO-A-95/24923    WO-A-96/10421
WO-A-97/06821    WO-A-97/26910

• BARRIOS ET AL.: "MYCOBACTERIAL HEAT-SHOCK PROTEINS AS CARRIER MOLECULES" EUR. J. IMMUNOL., vol. 22, 1992, pages 1365-1372, XP002061368
• SRIVASTAVA P K ET AL: "HEAT SHOCK PROTEIN-PEPTIDE COMPLEXES IN CANCER IMMUNOTHERAPY" CURRENT OPINION IN IMMUNOLOGY, vol. 6, 1994, pages 728-732, XP002037578
• LEVI AND ARNON: "SYNTHETIC RECOMBINANT INFLUENZA VACCINE INDUCES EFFICIENT LONG-TERM IMMUNITY AND CROSS-STRAIN PROTECTION" VACCINE, vol. 14, no. 1, January 1996, pages 85-92, XP002061369
• DENAGEL AND PIERCE: "HEAT SHOCK PROTEINS IN IMMUNE RESPONSE" CRITICAL REVIEWS IN IMMUNOLOGY, vol. 13, no. 1, 1993, pages 71-81, XP002061370

**Description**

BACKGROUND OF THE INVENTION

The viruses causing influenza have been arbitrarily named as influenza type A, B, and C. These types define antigenically distinct viruses. Each type has several distinct subtypes. Viruses within one type are genetically compatible in the sense that cells infected with two different subtypes can assemble mixed viruses containing components from both subtypes. Influenza viruses are classified as orthomyxoviruses. The viruses form particles of between 80 and 120 nm in diameter. Influenza viruses are enveloped viruses, i.e., their outer surface is derived from host cell membrane. Inserted in and protruding from the envelope are two major viral-encoded proteins, hemagglutinin (HA) and neuraminidase (NA). Influenza viruses are negative-stranded RNA viruses, containing a genome made up of 8 RNA segments of non-messenger RNA polarity. The genomic RNA segments are assembled in RNP complexes with virus-encoded nuclear protein (NP). Following infection of a host cell, genomic RNA segments are first transcribed into RNAs with messenger RNA polarity which are later reverse-transcribed to produce genomic RNA. The transcriptase activities responsible for these steps lacks proof-reading capability. Mistakes that are made during transcription and reverse transcription are therefore not repaired, resulting in a high frequency of mutation of the viral genome. While all viral genes are subject to the same mutational process, genes for external proteins HA and NA are particularly subject to strong selection processes that drive their evolution towards mutant forms that escape immune detection in their hosts. Hosts include not only humans but also animals such as chicken, turkey, swine and horse.

Influenza has traditionally been one of the leading causes of human death. The clinical signs of influenza are variable, ranging from asymptomatic to fatal infection. Typically, onset of illness is rapid and prostrating, and is almost invariably attended by cough, malaise, headache, and myalgia. Coryza, sore throat, and, less commonly, substernal pain also indicate that the primary site of infection is the respiratory system. Typically, however, fever and systemic symptoms predominate. Recovery typically is rapid. The severity of the disease is largely host-dependent and relates to age, physiological state and prior immunization by infection or immunization. A severe complication is pneumonia. Compromised individuals are prone to suffer secondary infections with bacterial pathogens that cause pneumonia. Most patients who die following influenza die with bacterial pneumonia. Minor antigenic variations in influenza virus types A and B occur yearly, causing regional epidemics. The yearly death rate from such yearly epidemics may approach 20,000 in the U.S. alone. At variable intervals between 10 and 30 years, global pandemics occur with death tolls far exceeding that of yearly epidemics. These pandemics are probably caused by genetic reassortment of components from human and animal influenza A viruses, resulting in new virus with a surface structure totally alien to human experience. The death toll of the 1918-19 pandemic killed about 500,000 Americans. (As a general reference: Joshua Lederberg, *Encyclopedia of Microbiology, 2*(D-L):505-520, Academic Press Inc., San Diego, CA 92101 (1992).

Presently licensed vaccines include inactivated purified virus. The vaccines are trivalent and include representative strains of the two prevalent A subtypes, H3N2 and H1Ni, and a single type B strain. Attenuated live virus vaccines have also been used with some success, particularly in the previous Soviet Union. Subunit vaccines have been developed containing HA and NA (split flu vaccine; Connaught Lab.). These vaccines are not completely effective in providing protective immunity. It is generally accepted that influenza vaccines generate protective immunity mainly by means of inducing antibody responses to the viral surface proteins HA and NA. This may explain why the vaccines are only incompletely effective; they are susceptible to continuous antigenic variation in these surface proteins.

The search for differences between tumor cells and normal cells has led to the isolation and characterization of a number of so-called tumor-associated antigens (Henderson, R.A. and Finn, O.J., *Advances in Immunology, 62*: 217-256 (1996)). These antigens are expressed by tumor cells but not at all or at least not in large amounts in fully differentiated cells. The sequences encoding these tumor antigens are either virus-derived or are normally present in the genome of the host. An example of a virus-derived tumor-associated antigen is the human papillomavirus transforming protein E7 present in most human cervical tumors. A typical host genome-derived tumor-associated antigen is gp 100, also referred to as pMel-17, that is expressed in many human melanomas. While tumor-associated antigens are known to induce a host immune response, the response is typically insufficient to be therapeutically effective. There is a need for approaches to stimulate this response.

Using monospecific cytotoxic T lymphocyte (CTL) clones, the expression of at least five tumor-associated antigens, termed A, B, C, D and E, has been identified in mouse P815 mastocytorna tumor cells. One of these antigens, termed P1A, expresses a single epitope that is recognized by CTL clones. Using a molecular approach, the gene for P1A was cloned and was found to be a nonmutated gene present in normal cells but transcribed and translated only in transformed cells (Van den Eynde *et al., J. Exp. Med., 173*:1373 (1991)). Further, by examination of variants of P815 cells that had lost P1A antigen expression, it was possible to identify the sequence of the MHC class I (L$^d$)-restricted minimal CTL epitope of P1A (Lethe, *et al., Eur. J. Immunol., 22*:2283 (1992)).

Barios *et al, EUR. J. Immunol.* (22: 1365-1372, 1992) discloses a conjugate of a peptide and a heat shock protein, the conjugate made according to standard practice, by mixing two proteins in the presence of glutaraldehyde, a crosslinking agent.

Srivastava and Udono, *Current Opin. Immunol.* (6: 728-232, 1994) discloses non-covalent complexes of HSPs and antigens isolated from tumors.

WO 94/29459 (Young) discloses a fusion protein which contains HSP70 and an HIV antigen.

WO 95/24923 (Srivastava *et al*) discloses stress protein peptide complexes.

WO 94/03208 (Cohen *et al*) discloses various combinations of a T-cell epitope-containing fragment of the human hsp60 protein and a poorly immunogenic antigen, namely a polysaccharide.

WO97/06821 relates to methods and compositions for inducing an immune response in a subject, wherein at least one heat shock protein in combination with one or more defined target antigens is administered to the subject.

WO97/26910 discloses a composition comprising tumor cells, into which a gene encoding a heat shock protein has been inserted, or tumor antigens which were bound to a heat shock protein. It will be appreciated that this document does not disclose a fusion protein.

Thus, a need exists for more effective vaccines against antigens-associated with viruses and tumors.

According to the present invention there is provided a fusion protein as defined in claim 1.

The stress protein for use in the present invention can be, for example, a mycobacterial stress protein (e.g., hsp65, hsp71) or a Stress protein having an amino acid sequence sufficiently homologous to the amino acid sequence of the mycobacterial stress protein to induce the immune response to the antigen in the mammal to whom it is administered. The vaccine for inducing a cell-mediated cytolytic immune response against an antigen in a mammal can also comprise a polynucleotide which encodes and directs expression of an antigen and a stress protein sequence in the mammal. The polynucleotide can express the antigen and stress protein as a fusion protein.

In one embodiment, the present invention relates to a vaccine for inducing a cell-mediated cytolytic immune response against an antigen of an influenza virus in a mammal comprising a polynucleotide which directs expression of the antigen of the influenza virus and a stress protein in the mammal. The antigen of the influenza virus which can be used in the present invention includes, for example, hemagglutinin, nucleoprotein, neuraminidase, M1, M2, PB1, PB2, PA and a combination thereof.

The present invention also relates to compositions comprising a stress protein and an antigen of an influenza virus as defined in claim 15. In an embodiment, the composition is a fusion protein (pET65MP/NP-B and pET65M/NP-D) comprising a stress protein fused to an antigen of the influenza virus.

The present invention also relates to use of the compositions for preventing or treating influenza virus in a mammal.

A vaccine for inducing a cell-mediated cytolytic immune response to a tumor-associated antigen in a mammal is also disclosed, the vaccine comprising a tumor-associated antigen linked to all or a portion of a stress protein or all or a portion of a protein having an amino acid sequence sufficiently homologous to the amino acid sequence of the stress protein to induce the immune response against the antigen. The antigen which can be used in the present invention comprises any mammalian tumor-associated antigen including those presently known in the art. It also includes fragments of these antigens that contain a CTL epitope.

Further disclosed is the vaccine for inducing a cell-mediated cytolytic immune response to a tumor-associated antigen in a mammal where the vaccine is a polynucleotide containing in expressible form sequences encoding a tumor-associated antigen and all or a portion of a stress protein or all or a portion of a protein having an amino acid sequence sufficiently homologous to the amino acid sequence of the stress protein to induce the immune response against the antigen.

Also disclosed is the vaccine for inducing a cell-mediated cytolytic immune respoxtse to a tumor-associated antigen in a mammal that can also be a polynucleotide encoding a recombinant fusion protein which includes a tumor-associated antigen and all or a portion of a stress protein or all or a portion of a protein having an amino acid sequence sufficiently, homologous to the amino acid sequence of the stress protein to induce the immune response against the antigen.

There is also disclosed vaccines for suppressing allergic immune responses to natural or artificial antigens (allergens) in a mammal, the vaccines including an allergen and all or a portion of a stress protein or all or a protion of a protein having an amino acid sequence sufficiently homologous to the amino acid sequence of the stress protein to suppress the allergic responses. Any allergen, regardless of whether it is peptidic or not, can be used.

Further disclosed is the vaccine for suppressing allergic immune responses to natural or artificial antigens (allergens) in a mammal is an allergen chemically conjugated to all or a portion of a stress protein or all or a portion of a protein having an amino acid sequence sufficiently homologous to the amino acid sequence of the stress protein to suppress the allergic responses.

In another disclosed, non-claimed embodiment, the vaccine for suppressing allergic immune responses to natural or artificial antigens (allergens) in a mammal is a recombinant fusion protein which includes an allergen and all or a portion of a stress protein or all or a portion of a protein having an amino acid sequence sufficiently homologous to the amino acid sequence of the stress protein to suppress the allergic responses.

In a further disclosed non-claimed embodiment, the vaccine for suppressing allergic immune responses to natural or artificial antigens (allergens) in a mammal is a polynucleotide containing in expressible form sequences encoding a peptidic allergen and all or a portion of a stress protein or all or a portion of a protein having an amino acid sequence sufficiently homologous to the amino acid sequence of the stress protein to suppress the allergic responses.

In yet another disclosed non-claimed embodiment, the vaccine for suppressing allergic immune responses to natural or artificial antigens (allergens) in a mammal can also be a polynucleotide encoding a recombinant fusion protein which includes a peptidic allergen and all or a portion of a stress protein or all or a portion of a protein having an amino acid sequences sufficiently homologous to the amino acid sequence of the stress protein to suppress the allergic responses.

A composition for suppressing a Th2 response to an antigen in a mammal is also disclosed comprising the antigen and all or a portion of a stress protein or all or a portion of a protein having an amino acid sequence sufficiently homologous to the amino acid sequence of the stress protein to suppress the Th2 response to the antigen. The composition can be a vaccine, conjugate or fusion protein.

BRIEF DESCRIPTION OF THE FIGURES

Figure 1 is a.graph of effector:target ratio versus % specific cell lysis demonstrating a cytolytic T cell (CTL) response in mice to a mixture comprising nucleoprotein (NP) peptide and heat shock protein 70 (hsp70).

Figure 2 is a graph of effector:target ratio versus % specific cell lysis demonstrating a CTL response in mice to a composition comprising a chemical conjugate of an NP peptide and hsp70.

Figure 3 is a schematic representation of the vector, pET65MP.

Figure 4A is a schematic representation of the vector, pET65MP/NP-B.

Figure 4B is a schematic representation of the vector, pET65MP/NP-D.

Figures 5A-5B are graphs of effector:target ratio versus % specific cell lysis demonstrating a CTL response in mice to the hsp-NP fusion protein, hsp65-NP.B, wherein the effector cells were restimulated in the absence of IL-2 (Figure 5A) and in the presence of IL-2 (Figure 5B).

Figures 6A-6B are graphs of effector:target ratio versus % specific cell lysis demonstrating a CTL response in mice to the hsp-NP fusion protein, hsp65-NP.D, wherein the effector cells were restimulated in the absence of IL-2 (Figure 6A) and in the presence of IL-2 (Figure 6B).

Figures 7A-7B are graphs of effector:target ratio versus % specific cell lysis demonstrating a CTL response in BALB/c mice immunized with an hsp-P1A fusion protein.

Figures 8A-8B are graphs of effector:target ratio versus % specific cell lysis demonstrating a CTL response in DBA/2 (H-2$^d$) mice immunized with an hsp-P1A fusion protein.

Figure 9 is a bar graph demonstrating that immunization with the hsp-tumor-associated antigen, hsp71-P1A, results in stimulation of CTL activity directed against cells displaying relevant MFIC class I-restricted epitopes.

DETAILED DESCRIPTION OF THE INVENTION

The present application discloses vaccines and compositions which induce an immune response to an antigen in a mammal (e.g., human) comprising an antigen (one or more) and all or a portion of a stress protein or heat shock protein (one or more) or all or a portion of a protein having an amino acid sequence sufficiently homologous to the stress protein to induce the immune response against the antigen. Particularly, vaccines and compositions which induce a cell mediated immune response in a mammal comprising an antigen (one or more) and all or a portion of a stress protein (one or more) or all or a portion of a protein having an amino acid sequence sufficiently homologous to the stress protein to induce the immune response against the antigen are disclosed.

The invention relates to compositions which induce an immune response to an influenza virus in a mammal comprising an antigen of the influenza virus and all or a portion of a stress protein or all or a portion of a protein having an amino acid sequence sufficiently homologous to the amino acid sequence of the stress protein to induce the immune response against the antigen as defined in claim 1. As described herein compositions comprising an influenza antigen (e.g., NP sequences including CTL epitopes) and at least one stress protein, in the form of a fusion protein containing influenza antigen and stress protein sequences, are effective in stimulating specific immune responses (e.g., cytolytic T cell (CTL) response, T cell helper response, B cell response) against the influenza antigen used in mammals. For example, as demonstrated in the examples, immunization of a host (vertebrate, such as a mammal) with the vaccines described herein can result in stimulation of specific CTL activity directed against cells displaying the influenza antigen (e.g., NP).

Vaccines that induce a cell-mediated immune response to tumor-associated antigens are also disclosed comprising a tumor-associated antigen suitable for immunization against a pre-existing tumor of a particular type or for prevention of the development of such tumor and all or a portion of a stress protein or all or a portion of a protein having an amino acid sequence sufficiently homologous to the amino acid sequence of the stress protein to induce the immune response against the antigen. Analogous to the previous embodiment, vaccines comprising at least one tumor-associated antigen and one stress protein, in the form of fusion proteins containing tumor-associated antigen and stress protein sequences, can stimulate cell-mediated cytolytic immune responses against the tumor-associated antigen in mammals. As demonstrated in the examples, a vaccine of this type, a fusion protein containing a minimal P1A mastocytoma antigen and a stress protein, induces a cell-mediated, cytolytic response against cells displaying the P1A antigen. Moreover, mammalian animals immunized with the vaccine are immune against a subsequent challenge with tumor cells expressing the P1A antigen.

In the present invention, the composition is comprised of two moieties: a stress protein and an antigen against which an immune response is desired. The two moieties form a single unit. Conjugation can be achieved by recombinant techniques. If recombinant techniques are used to link or connect the two moieties, the result is a recombinant fusion protein which includes the stress protein and the antigen in a single molecule. This makes it possible to produce and purify a single recombinant molecule in the vaccine production process. The stress protein can be conjugated to any influenza antigen against which the cell mediated, cytolytic immune response is desired or to a portion of the antigen sufficient to induce an immune response in an individual to whom it is administered.

As defined herein the term "vaccine" includes compositions which can be used as a prophylactic or a therapeutic vaccine. In one embodiment, the vaccine composition is one or more nucleic acids which encode the antigen and the stress protein. The present invention also relates to use of the compositions which are nucleic acids encoding a stress protein and/or the antigen for preventing or treating a disease or condition associated with or caused by the presence of an influenza virus. For example, the compositions describe herein can be used to induce an immune response against an influenza virus in a mammal not infected with the virus. In addition, the vaccines or compositions described herein can be used to induce an immune response against an influenza virus in a mammal infected with an influenza virus, and can result in amelioration or elimination of the disease state caused by the infecting influenza virus in the mammal. As used herein "induction of an immune response" means an increased immune response (more than undetectable or more than before); or a response that is superior to that achievable by immunization, under comparable conditions, with antigen alone.

As described herein, an antigen (one or more per stress protein) preferably is of a peptidic nature, i.e., it is a protein, polypeptide or peptide. In applications in which antigen and stress protein are admixed or chemically linked, the antigen can also be a carbohydrate, lipid, glycolipid or organic or inorganic molecule. As used herein an "antigen" includes peptides or polypeptides which comprises at least one CTL epitope. A CTL epitope is defined as either a class I-restricted T cell epitope or a class II-restricted T cell epitope. The antigen for use in the present invention can be isolated, purified (essentially pure), chemically synthesized or recombinantly produced. Other suitable antigens useful in the compositions of the present invention can be determined by those of skill in the art.

In the embodiment in which the vaccine or composition induces a cell-mediated, cytolytic immune response to an influenza virus, antigens of the influenza virus include but are not limited to peptides or polypeptides which comprises at least one B cell and/or T cell (e.g., T helper cell, cytolytic T cell) epitope. For example, an antigen of the influenza virus includes, but is not limited to hemagglutinin (HA, e.g., HA1, HA7), nucleoprotein (e.g., NP, such as NP-b and NP-D described in the examples), neuramidase (NA), M1, M2 PB1, PB2 and PA. Other antigens of an influenza virus which can be used in the compositions of the present invention can be determined by those of ordinary skill in the art.

In the event that the vaccine or composition induces a cell-mediated, cytolytic immune response against a tumor-associated antigen, antigens include, but are not limited to, MAGE1, MAGE3, BAGE and GAGE. These proteins are normally expressed in testis.

Ectopic expression gives rise to a variety of tumors including melanocnas. Also included in this list are melanocyte differentiation antigens Tyrosinase, MART-1/MELAN-1 and gp 100/pMe117 as well as tyrosinase-related protein pg75 and MUM-1, all of which are associated with melanomas. Other useful tumor-associated antigens are HER2/neu found in breast and ovarian tumors, MUC-1 found in epithelial cell tumors, and human papillomavirus proteins E6 and E7 which are associated strongly with cervical tumors. Additional antigens include GnT-V, beta-catenin, CDK4 and p15. All these tumor-associated antigens are recognized by T cells. (Wang, R.-F. and Rosenberg, S.A., *Journal of Leukocyte Biology, 60*:296-309 (1996); Houghton; A.N., *J. Exp. Med., 180*:1-4 (1994); Henderson, R.A. and Finn, O.J., *Advances of Immunology, 62*:217-256) .

The important players in allergic (atopic) and asthmatic disease are IgE and local inflammatory reactions dominated by the infiltration of eosinophils.

Pulmonary hyperreactivity to nonspecific stimuli due to chronic inflammation is the modern definition of asthma. This inflammation may be cause by abnormal allergic responses to natural or artificial antigens mediated by IgE and leads to a chronic cellular infiltration of granular cells called eosinophils. The release of mediators from resident mast cells and recruited basophils and eosinophils is thought to be the cause of the inflammation and subsequent hyperreactivity. In man, as in other species, any inflammatory reaction produces local hyperreactivity. However, in asthma the inflammation is chronic, leading to life-threatening hyperreactivity unless treated appropriately. Current treatment includes the use of corticosteroids to reduce the inflammation and bronchodilators such as albuterol (beta agonists) for prompt symptomatic relief.

In humans as in mice, two distinct patterns of cytokine secretion have been defined among CD4+ helper T cell clones (del Prete, G., *Allergy*, 47:450-455 (1992)).

Human type 1 helper (Th1) but not type 2 helper (Th2) cells produce interleukin-2 (IL-2), gamma interferon and tumor necrosis factor beta. Th2 cells but not Th1 cells secrete IL-4 and IL-5 but not IL-2 or gamma interferon.

Other cytokines such as IL-3, IL-6, GM-CSF or tumor necrosis factor alpha are produced by both Th1 and Th2 cells. The different cytokine patterns are associated with different functions. In general, Th2 cells provide an excellent helper function for B cell antibody production, particularly of the IgE class. Th1 cells are responsible for delayed hypersensitivity reactions and are cytolytic for autologous antigen presenting cells including B cells. Most allergen- or helminth-antigen specific human CD4+ T cell clones exhibit a Th2 phenotype while most clones specific for bacterial antigens show a Th1 profile. Allergen specific Th2 cells seem to play a crucial role in atopy. These cells induce IgE production via IL-4 and favor the proliferation, differentiation and activation of eosinophils via IL-5. In addition, Th2 derived IL-3, IL-4 and IL-13 are mast cell growth factors that act in synergy, at least *in vitro*. There is evidence that allergen-specific Th2 cells are selectively enriched in tissues affected by allergic inflammation such as the bronchial mucosa of humans with allergic asthma.

With the increasing use of antibiotics in early childhood in the developed world, the incidence of and deaths due to (allergic) asthma are rising. The following discussion is using this information that links increased incidence and severity of allergic reactions to a lack of exposure and T cell memory for bacterial proteins including stress proteins to support the notion that deliberate exposure to bacterial antigens including stress proteins will dampen allergic responses.

Many scientists believe that the development of resistance or sensitivity to environmental antigens depends on the nature of immunological memory generated during early antigen encounters in infancy and early childhood (Holt P.G., *Toxicol Lett. ,* 86:205-210). This process appears to be antigen driven. Selection is for specific Th1 versus Th2 like memory cells within individual immune responses to inhaled antigens, a process which occurs in the regional lymph nodes draining the conducting airways. This selection appears to be regulated by a variety of cytokines produced by antigen specific CD4+ and CD8+ T cells. This T cell selection process can theoretically be influenced by infectious agents: infections in the airway mucosa may mobilize and activate local tissue (alveolar) macrophages which migrate to the regional lymph nodes and secrete Th2 inhibitory cytokines such as IL-12 and alpha-interferon. In addition, they may add to the gamma-interferon levels in the milieu through activation of natural killer cells. The net result is the production of CTLs (which are predominantly CD8+ cells).

Gamma-interferon inhibits the generation of Th2 cells and therefore production of IL-4 and IL-5, cytokines crucial for the generation of humoral (IgE) and cellular (eosinophils, basophils and mast cells) allergic responses (Anderson, G.P. and Coyle, A.J., Trends *Pharmacol. Sci.*, 15:324-332 (1995); Stam, W.B., van Oosterhout, A.J. and Nijkamp, F.P., *Life Sci.*, *53*:1921-1934 (19939)).

In mammals, stress proteins have been shown to induce humoral as well as cellular immune responses. As shown in the examples herein, when soluble antigen mixed with, chemically conjugated to or fused to a stress protein is administered to a mammal, cell-mediated cytolytic immune responses are substantially enhanced. These responses are largely due to CD8+ T cells. Therefore, a comparison of the CD4+ responses to antigens by themselves to those mixed with or coupled to stress proteins give the predicted profile: soluble antigens mixed with or linked to stress proteins yield a high proportion of CTLs (mainly CD8+ T cells) which are a measure of stimulation of the Th1 pathway described before because these CTLs arose as a result of the induction of antigen specific T cells of the Th1 type. These Th1 cells produce gamma-interferon, which cytokine inhibits Th2 cells. Therefore, the Th2 cytokines IL-4 and IL-5 are no longer available to support the production of IgE and eosinophils. With decreasing titer of IgE, direct antigenic stimulation of mast and basophil cells will decline. In addition, decreased IL-5 production will lead to decreased production, differentiation and activation of eosinophils. This pattern will cause decreased inflammation of the involved tissue and result in less hyperreactive (asthmatic) events.

Therefore, administration of mixtures of known allergenic antigens (allergens) and stress proteins or compositions containing allergens chemically linked to or fused to stress proteins should influence the Th1 to Th2 ratio in atopic patients, restoring a more normal balance and leading to decreased allergy or asthma. Stress proteins used in such compositions are preferably of bacterial or mycoplasmic origin. Allergens used in allergen-stress protein fusion proteins are necessarily of a peptidic nature; nonpeptidic allergens can be used in conjugates containing an allergen and a stress protein or a mixtures of allergen and stress protein. Nonlimiting examples for allergens include Fel d 1 (cat); Amb a 1 (antigen E), Amb a 2 (antigen K) (ragweed); Der f 2, Der p 1, Der p 9, Der f 1 (mites); Bla g 1, Bla g 2 (cockroach); Bet v 1 (birch); Rat n 1 (rat); Cha o 1 (Japanese cypress); Hev b 5 (latex); gp40 (mountain cedar). For a reasonably comprehensive list of allergens up to the time of publication, see King, T.P. *et al.*, *Int. Arch. Allergy Immunol., 105*: 224-233 (1994).

When compositions containing covalently linked or admixed allergen and stress protein are administered by a suitable route such as subcutaneous or intramuscular injection or even given by inhalation to a patient in need of treatment for hypersensitivity reactions, they should produce a decrease in allergic symptoms as measured by the classic hyperreactivity test in asthma, for example. After treatment, the patient will exhibit less nonspecific reactivity. In asthmatics, or in animal models of asthma, hyperreactivity is measured by determining the doses of inhaled methacholine that induce a bronchoconstrictive response. Mammals with chronic inflammatory conditions which lead to hyperreactivity will exhibit greater sensitivity to methacholine challenge. They will bronchoconstrict at lower doses than "normal" mammals. After treatment with the appropriate stress protein-containing composition, the dose response to methacholine would shift to less sensitive.

Any suitable stress protein (heat shock protein (hsp)) can be used in the compositions of the present invention. For example, as described in the examples, hsp65 and/or hsp71 can be used. Turning to stress proteins generally, cells respond to a stressor (typically heat shock treatment) by increasing the expression of a group of genes commonly referred to as stress, or heat shock, genes. Heat shock treatment involves exposure of cells or organisms to temperatures that are one to several degrees Celsius above the temperature to which the cells are adapted. In coordination with the induction of such genes, the levels of corresponding stress proteins increase in stressed cells. As used herein, a "stress protein," also known as a "heat shock protein" or "Hsp," is a protein that is encoded by a stress gene, and is therefore typically produced in significantly greater amounts upon the contact or exposure of the stressor to the organism. A "stress gene", also known as "heat shock gene" is used herein as a gene that is activated due to the contact or exposure of an organism (containing the gene) to a stressor, such as heat shock or glucose deprivation or addition. "Stress gene" also includes homologous genes within known stress gene families, such as certain genes within the Hsp70 and Hsp90 stress gene families. Each of the terms stress gene and stress protein as used in the present specification may be inclusive of the other, unless the context indicates otherwise.

The stress proteins for may be isolated stress proteins, which means that the stress proteins have been selected and separated from the host cell in which they were produced. Such isolation can be carried out as described herein and using routine methods of protein isolation known in the art. (Maniatis *et al.*, *Molecular Cloning, A Laboratory Manual,* Cold Spring Harbor Laboratory, Cold Spring Harbor, N.Y., 1982; Sambrook et al., *Molecular Cloning: A Laboratory Manual*, 2d Ed., Cold Spring Harbor Laboratory Press, 1989)). The isolated stress protein may also, further, be purified (essentially pure) in accordance with the methods, particularly the detergent purification methods.

In bacteria, the predominant stress proteins are proteins with molecular sizes of about 70 and 60 kDa, respectively, that are referred to as Hsp70 and Hsp60, respectively. These and other specific stress proteins and the genes encoding them are discussed further below. In bacteria, Hsp70 and Hsp60 typically represent about 1-3% of cell protein based on the staining pattern using sodium dodecyl sulfate polyacrylamide gel electrophoresis and the stain coomassie blue, but accumulate to levels as high as 25% under stressful conditions. Stress proteins appear to participate in important cellular processes such as protein synthesis, intracellular trafficking, and assembly and disassembly of protein complexes. It appears that the increased amounts of stress proteins synthesized during stress serve primarily to minimize the consequences of induced protein unfolding. Indeed, the preexposure of cells to mildly stressful conditions that induce the synthesis of stress proteins affords protection to the cells from the deleterious effects of a subsequent more extreme stress.

The major stress proteins appear to be expressed in every organism and tissue type examined so far. Also, it appears that stress proteins represent the most highly conserved group of proteins identified to date. For example, when stress proteins in widely diverse organisms are compared, Hsp90 and Hsp70 exhibit 50% or higher identity at the amino acid level and share many similarities at nonidentical positions.

The genes encoding stress proteins may be present in a single copy or in multiple, non-identical copies in the genome of a cell or organism. For example, the human genome has been shown to contain at least one copy of an Hsp100 gene, at least two different Hsp90 genes, up to ten Hsp70 genes of which at least several are non-identical copies, several T complex genes (Tcp genes) and at least one gene encoding the related mitochondrial protein Hsp60, as well as at least three copies of small Hsp genes encoding proteins in the 20-30 kDa range of molecular size. In most groups of stress genes there is at least one gene whose expression level is relatively high and is either entirely constitutive or only mildly heat shock-inducible. Furthermore, several groups of stress genes include members that are not up-regulated by heat but by other cues such as increased calcium levels, etc.

The stress proteins, particularly Hsp70, Hsp60, Hsp20-30 and Hsp10, are among the major determinants recognized by the host immune system in the immune response to infection by *Mycobacterium tuberculosis* and *Mycobacterium leprae.* Young, R.A., and Elliott, T.J., Stress Proteins, Infection, And Immune Surveillance, *Cell 50:*5-8, (1989). Further, some rat arthritogenic T-cells recognize Hsp60 epitopes. Van Eden, W., Thole, J., van der Zee, R., Noordzij, A., van Embden, J., Hensen, E., and Cohen, I., *Nature 331:*171-173, (1988) . However, individuals, including healthy individuals, with no history of mycobacterial infection or autoimmune disease also carry T-cells that recognize both bacterial and human Hsp60 epitopes; a considerable fraction of T-cells in healthy individuals that are characterized by expression of the gamma-delta T-cell receptor recognize both self and foreign stress proteins. O'Brien, R., Happ, M.,

Dallas, A., Palmer, E. Kubo, R., and Born, W., *Cell 57*:664-674 (1989). Thus, individuals, even healthy individuals possess T-cell populations that recognize both foreign and self stress protein epitopes.

This system of recognizing stress protein epitopes constitutes an "early defense system" against invading organisms. The system may be maintained by frequent stimulation by bacteria and viruses that cause the host cells to upregulate their own stress genes. However, the presence of autoreactive T-cells is compatible with normal health and does not cause autoimmune disease; this also demonstrates the safety of stress proteins within an individual. The safety of stress proteins is additionally demonstrated by the success and relative safety of BCG (Bacille Calmette Guerin, a strain of *Mycobacterium bovis*) vaccinations, which induce an immune response against stress proteins that is also protective against *Mycobacteriurn tuberculosis.*

Stress genes and proteins for use in the present invention are those well known in the art and include, for example, Hsp100-200, Hsp100, Hsp90, Lon, Hsp70, Hsp60, TF55, Hsp40, FKBPs, cyclophilins, Hsp20-30, C1pP, GrpE, Hsp10, ubiquitin, calnexin, and protein disulfide isomerases. Macario, A.J.L., Cold Spring Harbor Laboratory Res. 25: 59-70, 1995; Parsell, D.A., & Lindquist, S., *Ann. Rev. Genet. 27*:437-496 (1993); U.S. Patent No. 5,232,833 (Sanders *et al.*). A particular group of stress proteins includes Hsp90, Hsp70, Hsp60, Hsp20-30, and ubiquitin, further preferably Hsp70 and Hsp60.

A stress protein in the methods and compositions of the present invention is preferably selected from extracellularly antigen-presenting stress proteins or from stress proteins that are processed and the resulting peptide fragments are presented on the surface of the cell, such that it is an extracellularly antigen-presenting protein. Additionally, a selected stress gene or protein for use in the present invention is preferably selected such that the stress gene or protein is unregulated pursuant to one or more forms of stress in at least one expression, preferably a bacterium or a human. Further preferably, the selected stress genes or proteins are unregulated in humans, including by stressors such as those described above or transformation.

Hsp100-200 examples include Grp170 (for glucose-regulated protein), Grp170 resides in the lumen of the ER, in the pre-golgi compartment, and may play a role in immunoglobulin folding and assembly.

Hsp100 examples include mammalian Hsp110, yeast Hsp 104, c1pA, c1pB, c1pC, c1pX and c1pY. Yeast Hsp104 and *E. coli* clpA, form hexameric and *E. coil* c1pB, tetrameric particles whose assembly appears to require adenine nucleotide binding. Clp protease provides a 750 kDa heterooligomer composed of ClpP (a proteolytic subunit) and of C1pA. C1pB-Y are structurally related to C1pA, although unlike C1pA they do not appear to complex with C1pP.

Hsp90 examples include HtpG in *E. coli*, Hsp83 and Hsc83 yeast, and Hsp90$\alpha$, Hsp90$\beta$ and Grp94 in humans. Hsp90 binds groups of proteins, which proteins are typically cellular regulatory molecules such as steroid hormone receptors (*e.g.*, glucocorticoids, estrogen, progesterone, and testosterone), transcription factors and protein kinases that play a role in signal transduction mechanisms. Hsp90 proteins also participate in the formation of large, abundant protein complexes that include other stress proteins.

Lon is a tetrameric protein functioning as an ATP-dependent protease degrading non-native proteins in *E. coli*.

Hsp70 examples include Hsp72 and Hsp73 from mammalian cells, DnaK from bacteria, particularly mycobacteria such as *Mycobacterium leprae*, *Mycobacterium tuberculosis*, and *Mycobacterium bovis* (such as Bacille-Calmette Guerin), DnaK from *Escherichia coli,* yeast, and other prokaryotes, and BiP and Grp78.

Hsp70 is capable of specifically binding ATP as well as unfolded polypeptides and peptides, thereby participating in protein folding and unfolding as well as in the assembly and disassembly of protein complexes.

Hsp60 examples include Hsp65 from mycobacteria. Bacterial Hsp60 also commonly known as GroEL, such as the GroEL from *E. coli*. Hsp60 forms large homooligomeric complexes, and appears to play a key role in protein folding. Hsp60 homologues are present in eukaryotic mitochondria and chloroplasts.

TF55 examples include Tcpl, TRiC and thermosome. The proteins are typically occur in the cytoplasm of eukaryotes and some archaebacteria, and form multi-membered rings, promoting protein folding. They are also weakly homologous to Hsp60.

Hsp40 examples include DnaJ from prokaryotes such as *E. coli,* and mycobacteria and HSJ1, HDJ1 and Hsp40. Hsp40 plays a role as a molecular chaperone in protein synthesis, thermotolerance and DNA replication, among other cellular activities.

FKBPs examples include FKBP12, FKBP13, FKBP25, and FKBP59, Fpr1 and Nep1. The proteins typically have peptidyl-prolyl isomerase activity and interact with immunosuppressants such as FK506 and rapamycin. The proteins are typically found in the cytoplasm and the endoplasmic reticulum.

Cyclophilin examples include cyclophilins A, B and C. The proteins have peptidyl-prolyl isomerase activity and interact with the immunosuppressant cyclosporin A. The protein cyclosporin A binds calcineurin (a protein phosphatase). Hsp20-30 examples include $\alpha$-crystallin, and Hsp20-30 is also referred to as small Hsp. Hsp20-30 is typically found in large homooligomeric complexes or, possibly, also heterooligomeric complexes where an organism or cell type expresses several different types of small Hsps. Hsp20-30 interacts with cytoskeletal structures, and may play a regulatory role in the polymerization/depolymerization of actin. Hsp20-30 is rapidly phosphorylated upon stress or exposure of resting cells to growth factors.

ClpP is an *E. coli* protease involved in degradation of abnormal proteins. Homologues of ClpP are found in chloroplasts. C1pP forms a heterooligomeric complex with ClpA.

GrpE is an *E. coli* protein of about 20 kDa that is involved in both the rescue of stress-damaged proteins as well as the degradation of damaged proteins. GrpE plays a role in the regulation of stress gene expression in *E. coli.* HsplO examples include GroES and CpnlO. HsplO is typically found in *E. coli* and in mitochondria and chloroplasts of eukaryotic cells. HsplO forms a seven-membered ring that associates with Hsp60 oligomers. HsplO is also involved in protein folding.

Ubiquitin has been found to bind proteins in coordination with the proteolytic removal of the proteins by ATP-dependent cytosolic proteases.

In particular embodiments, the stress proteins of the present invention are obtained from enterobacteria, mycobacteria (particularly *M. leprae. M. tuberculosis* and *M. bovis, E. coli,* yeast, *Drosophila,* vertebrates, avians, chickens, mammals, rats, mice, primates, or humans.

The stress proteins may be in the form of acidic or basic salts, or in neutral form. In addition, individual amino acid residues may be modified by oxidation or reduction. Due to code degeneracy, for example, there may be considerable variation in nucleotide sequences encoding the same amino acid sequence. The fusion proteins of the present invention may also contain stress protein fragments obtained from stress proteins, provided such fragments include the conformational epitopes involved with enhancing the immune response to the chosen antigen. Stress protein fragments may be obtained by fragmentation using proteinases, or by recombinant methods, such as the expression of a portion of a stress protein-encoding nucleotide sequence (either alone or as fusions with another protein). Peptides may also be produced by such methods, or by chemical synthesis. The stress protein is fused to a second protein, which may or may not be a stress protein. The stress proteins may include mutations introduced at particular loci by a variety of known techniques. *See, e.g.,* Sambrook *et al., Molecular Cloning: 4 Laboratory Manual,* 2d Ed., Cold Spring Harbor Laboratory Press, 1989; Drinkwater and Klinedinst, *PNAS* 83:3402-3406, 1986; Liao and Wise *Gene* 88:107-111, 1990'); Horwitz *et al.,. Genome* 3:112-117, 1989.

The term "sufficiently homologous to the amino acid sequence of the stress protein" means that the amino acid sequence of the protein or polypeptide will generally show at least 40% identity with the stress protein amino acid sequence; in some cases, the amino acid sequence of a functional equivalent exhibits approximately 50% identity with the amino acid sequence of the stress protein.

Methods of identifying a gene or a protein under consideration as a stress gene or protein are well known in the art. For example, the conservation of the genes and proteins of a particular stress protein group permits comparison of the nucleotide or amino acid sequence of the gene/protein under consideration with well known stress genes such as DnaK, GroEL or DnaJ, *e.g.,* by nucleic acid hybridization or nucleic acid or amino acid sequencing followed by computer comparison analysis. Voellmy, R., *et al., PNAS 82:4949-4953* (1985). Alternatively, an assay may be used to identify and/or discriminate between essential structural features and/or functional properties of a selected stress protein. For example, an expression library may be screened using anti-Hsp antibodies and other assays well known in the art. Antibodies: *A Laboratory Manual* Harlow and Lane (eds.), Cold Spring Harbor Laboratory Press, (1988). In addition, the biological activity of a given stress protein group may be exploited. Guidon, P.T., and Hightower, L.E., *Biochem.*, 25: 3231-3239 (1986). For example, Hsp70 is capable of specifically binding ATP as well as unfolded polypeptides and peptides in the assembly of protein complexes. Thus, mixing a protein under consideration with a sample comprising appropriate polypeptides, peptides, or ATP, followed by determination of the presence or absence of production of protein-protein or protein-nucleic acid complexes indicates the apparent presence or absence of an Hsp70 gene or protein, which presence or absence can be confirmed utilizing other assays such as antibody-based assays.

An effective dosage of the stress proteins to elicit specific cellular and humoral immunity to stress proteins, or to substances conjugated to the stress proteins, such as proteins or oligosaccharides, is in the range of 0.1 to 1000 ug hsp per injection, depending on the individual to whom the stress protein is being administered (Lussow, A.R., *et al., Eur. J. Immun.*, 21:2297-2302 (1991); Barrios, C. et al., *Eur. J. Immun.,* 22:1365-1372 (1992)). The appropriate dosage of the stress protein for each individual will be determined by taking into consideration, for example, the particular stress protein being administered, the type of individual to whom the stress protein is being administered, the age and size of the individual, the condition being treated or prevented and the severity of the condition. Those skilled in the art will be able to determine using no more than routine experimentation, the appropriate dosage to administer to an individual.

The stress protein, stress protein portion, stress protein functional equivalent and the antigen to which the stress protein is admixed, fused or conjugated, present in the vaccine can be produced or obtained using known techniques. For example, the stress protein and/or the antigen of influenza virus can be obtained (isolated) from a source in which it occurs in nature, can be produced by cloning and expressing a gene encoding the desired stress protein or the antigen or can be synthesized chemically or mechanically.

The compositions described herein can be used to induce an immune response against a variety of pathogens (e.g., bacteria, virus, parasite). The composition comprising an antigen of the influenza virus and all or a portion of one or more stress proteins or all or a portion of a protein having an amino acid sequence sufficiently homologous to the amino acid sequence of the stress protein to induce the immune response against the antigen can be used to induce an immune response to an influenza virus in any vertebrate (e.g., mammals, fowl) susceptible to an influenza virus. For example, the compositions can be used to induce an immune response against an influenza virus in primates (e.g., humans), horses, swine, turkeys and chickens.

The compositions described herein can be administered to a host in a variety of ways. The routes of administration include intradermal, transdermal (e.g., slow release polymers), intramuscular, intraperitoneal, intravenous, subcutaneous, oral, epidural and intranasal routes. Any other convenient route of administration can be used, for example, infusion or bolus injection, or absorption through epithelial or mucocutaneous linings. In addition, the compositions described herein can be administered together with other components or biologically active agents (e.g., alum), pharmaceutically acceptable surfactants (e.g., glycerides), excipients (e.g., lactose), carriers, diluents and vehicles.

Further, the stress protein and/or peptidic antigen can be administered by *in vivo* expression of polynucleotides encoding such into a mammalian subject.

That is, a vector can be used to deliver nucleic acid(s) encoding an antigen and a stress protein or a nucleic acid encoding a fusion protein containing antigen and stress protein sequences. For example, the stress protein and/or the antigen can be administered to host (mammal) using live vectors wherein the live vectors containing stress protein and antigen nucleic acid sequences are administered under conditions in which the antigen and/or the stress protein are expressed *in vivo.* For example, a mammal can be injected with a vector which encodes and expresses an antigen *in vivo* in combination with a stress protein in protein or peptide form, or in combination with a vector which codes for and expresses a stress protein *in vivo.*

Alternatively, a host can be injected with a vector which encodes and expresses stress protein *in vivo* in combination with an antigen in peptide or protein form, or in combination with a vector which encodes and expresses an antigen *in vivo.* A single vector containing the sequences encoding a protein (peptide) antigen can also be used for the compositions of the present invention.

Several expression vector systems are available commercially or can be reproduced according to recombinant DNA and cell culture techniques. For example, vector systems such as the yeast or vaccinia virus expression systems, or virus vectors can be used (Kaufman, R. J. , *A J. of Meth. in Cell and Molec. Biol., 2*:221-236 (1990)). Other techniques using naked plasmids or DNA, and cloned genes encapsidated in targeted liposomes or in erythrocytes ghosts, can be used to introduce the stress protein and/or antigen polynucleotides into the host (Freidman, T., *Science,* 244:1275-1281 (199); Rabinovich, N.R., *et al.,* Science, *265*:1401-1404 (1994)). The construction of expression vectors and the transfer of vectors and nucleic acids into various host cells can be accomplished using genetic engineering techniques, as described in manuals like *Molecular Cloning* and *Current Protocols in Molecular Biology,* which are hereby incorporated by reference, or by using commercially available kits (Sambrook, J., *et al.*, *Molecular Cloning,* Cold Spring Harbor Press, 1989; Ausubel, F.M., et *al.*, *Current Protocols in Molecular Biology,* Greene Publishing Associates and Wiley-Interscience, 1989)).

The amount of stress protein and/or antigen in the compositions of the present invention is an amount which produces an effective immunostimulatory response in the host (vertebrate such as mammal). An effective amount is an amount such that when administered, it results in an enhanced immune response relative to the immune response when not administered. That is, an effective amount is an amount that provides a more pronounced immune response than similar amounts of the antigen or the stress proteins alone. In addition, the amount of stress protein and/or antigen administered to the host will vary depending on a variety of factors, including the antigen employed, the size, age, body weight, general health, sex, and diet of the host, and the time of administration, duration or particular qualities of the influenza virus. Adjustment and manipulation of established dose ranges are well within the ability of those skilled in the art. For example, the amount of stress protein and antigen can be from about 100 ug to about 1 g, preferably about 1 mg to about 1g, and from about 1 mg to about 100 mg.

The present invention teaches that the presence of a stress protein greatly stimulates the cell-mediated cytolytic response to an antigen. Although tumor-associated antigens have been identified, the immune responses against these antigens alone are not therapeutically effective. Enhancement of the cellular response against these antigens by means of coadministration of a stress protein, either in a mixture or linked to antigen, is beneficial in cancer therapy. This expectation is supported by the observation detailed in the examples that a composition of the present invention immunizes a mammalian animal against a subsequent tumor challenge. Enhancement of the cell-mediated, cytolytic response against an antigen is predicted to result in the downregulation of a preexisting (Th2-mediated) humoral response against the same antigen. Finally, T cell-mediated immunity is also considered to be an important element in the mammalian host's defense against infections caused by viruses, protozoa and certain intracellular bacteria such as mycobacteria. As is shown in the examples, compositions (mixtures, conjugates and fusion proteins) including a stress protein and an influenza virus antigen are effective in eliciting a substantial cell-mediated cytolytic response against mammalian cells expressing the viral antigen.

The present invention is illustrated by the following examples, which are not intended to be limiting in any way and of which examples 1-3 and 5-6 fall outside the scope of the claimed invention.

EXAMPLES

Example 1: Isolation of recombinant stress proteins

A. Recombinant Mycobacterial Hsp70. Plasmid Y3111 contains an *M. tuberculosis* Hsp70 gene functionally inserted between expression control sequences. (Mehlert, A. and Young, D.B., *Mol. Microbiol. , 3*:125-130 (1989). *E. coli* strain CG2027 (obtained from C. Georgopoulos, University of Geneva, Switzerland) containing a truncated Hsp70 gene was transformed with plasmid Y3111 by standard procedures. (Maniatis, *et al., Molecular Cloning, A Laboratory Manual,* Cold Spring Harbor Laboratory, Cold Spring Harbor, N.Y. (1982)).

Bacteria containing plasmid Y3111 were grown overnight in 2xYT medium (20 g Tryptone, 10 g yeast extract, 10 g NaCl per liter) containing 100 microgram/ml ampicillin at 37°C, with agitation (250 rpm). A 10% glycerol stock was prepared from this culture and was stored at -70°C. Several scrapings from the frozen glycerol stock were used to inoculate a large culture that was incubated as before for about 48 h. When the optical density at 590 nm reached 2.5 to 3.5, cells were collected by centrifugation.

The following steps were performed at 4°C. Cell pellets were resuspended in 3 ml per gram of lysis buffer. The composition of lysis buffer was 10 mM Tris-HCl, 2 mM ethylenediamine tetraacetate (EDTA), 5 mM beta-mercaptoethanol, 10 microgram/ml aprotinin, 10 microgram/ml leupeptin, and 1 microgram/ml pepstatin. Lysozyme was added to the cell suspension to a final concentration of 0.14 mg/ml. The suspension was then frozen at -70°C.

The cell suspension was thawed, and cells were broken by sonication. Sonicates were subjected to centrifugation at 17,000 rpm for 30 min. (JA-17 rotor, Beckmann). Solid $(NH_4)_2SO4$ was added to the supernatant solution until that solution was 65% saturated with $(NH_4)_2SO4$. After a 30 min. incubation, the mixture was centrifuged as before. The pellet was dissolved in Q SEPHAROSE buffer A. To this solution were added 10 microgram/ml aprotinin, 10 microgram/ml leupeptin, and 1 microgram/ml pepstatin, and the solution was dialyzed overnight against 65 volumes of Q SEPHAROSE buffer A. Q SEPHAROSE buffer A contained 30 mM Tris-HCl (pH 7.5), 1 mM EDTA, 5mM beta-mercaptoethanol. The dialyzed solution was clarified by centrifugation as described before.

Dialyzed solution was applied to a Q SEPHAROSE column (Pharmacia) equilibrated in Q SEPHAROSE buffer A. The column was washed with 2 volumes of the same buffer. Elution was with a 0 to 600 mM NaCl gradient. Fractions were tested by SDS-PAGE and staining with Coomassie Blue for the presence of a major 71 kDa polypeptide (i.e., the recombinant *M. tuberculosis* Hsp70 protein). Fractions containing the polypeptide were pooled, and the pool was brought to 65% saturation by the addition of solid $(NH_4)_2SO_4$. The mixture was centrifuged as described before, the pellet was dissolved in ATP Start buffer (50 mM Tris-HCl (pH 8.0), 20 mM NaCl, 5mM $MgCl_2$, 15 mM beta-mercaptoethanol and 0.1 mM EDTA), and the resulting protein solution dialyzed overnight against 65 volumes of the same buffer and clarified by centrifugation.

The dialyzed protein solution was then applied to an ATP-agarose column (Fluka) equilibrated in ATP Start buffer. The column was washed with 1 column volume of ATP Start buffer with 1 M NaCl. Elution was achieved with ATP Start buffer supplemented with 10mM ATP. The eluate was brought to 65% saturation with $(NH_4)_2SO4$, and precipitated protein was collected as described before. The centrifugation pellet was dissolved in and dialyzed against 200 volumes of Blue SEPHAROSE buffer (30 mM Tris-HCl (pH 7.5), 5mM $MgCl_2$, 5mM beta-mercaptoethanol).

The dialyzed protein solution from the last step was applied to a Blue SEPHAROSE column (Pharmacia) equilibrated Blue SEPHAROSE buffer. The column was washed with 1.5 column volumes of the same buffer. The flow-through and was fractions were collected as a single pool.

The purity of the final preparation was assessed by SDS-PAGE and Coomassie Blue staining, by western blot analysis (Maniatis, *et al.*, *Molecular Cloning, A Laboratory manual,* Cold Spring Harbor Laboratory, Cold Spring Harbor, NY (1982); (see Sambrook *et al., Molecular Cloning: A Laboratory manual, 2nd ed.*, Cold Spring Harbor Laboratory press, NY (1989)) using mouse monoclonal antibodies specific for mycobacterial Hsp70 and *E. coli* Hsp70, respectively, and by assays of ATPase activity. Preparations are typically more than 90% pure based on the staining pattern of the preparation in coomassie blue stained gels, and preferably more than 95% pure, and contained less than 1% of *E. coli* Hsp60 and no detectable *E. coli* Hsp70.

B. Mycobacterial Hsp60

Plasmid RIB1300 contains an *M. bovis* BCG Hsp60 gene functionally inserted between expression control sequences. (Thole, J.E.R., *et al.*, *J. Exp. Med.,* 178:343-348 (1993). *E. coli* strain M1546 was transformed with plasmid RIB1300 (Thole, J.E.R., *supra.)* using standard procedures. Maniatis, *et al.*, *Molecular Cloning, A Laboratory Manual,* Cold Spring Harbor laboratory, Cold Spring Harbor, NY (1982).

An inoculum of bacteria containing plasmid RIB1300 was grown to saturation in NCZYM medium (10 g N-Z Amine A, 5g Bacto yeast extract, 1g Casamino acids, 5g NaCl, 2g $(NH_4)_2SO_4$-$7H_2O$) per liter) containing 200 microgram/ml of ampicillin at 28°C and under agitation. This culture was used to inoculate a larger culture which was grown under the same conditions as the inoculum culture until the optical density of the culture was between 0.3 to 0.6 at an optical density of 590 nm. Production of the recombinant protein was initiated by rapidly raising the temperature of the culture to 42°C by incubation in a hot water bath. The culture was maintained at this temperature for 3h. The bacteria were then collected by centrifugation and resuspended in 6 volumes per weight of bacterial pellet of lysis buffer. Lysis buffer contained 10 mM Tris-JCL (pH 8.0), 10 mM ethylenediamine tetraacetate (EDTA), 0.1 mM PMSF and 0.1% RIVM BA (0.104 g 4-amino-benzamidine-2HCl, 0.066 g epsilon-amino caproic acid per 50 ml). Lysozyme was added to a concentration of 0.1 mg/ml, and the suspension was frozen at -70°C.

The bacterial suspension was thawed and placed at 4°C. The following operations were at this temperature. Complete lysis of bacteria was achieved by sonication. The sonicate was centrifuged at 17,000 rpm for 30 min in a JA-17 rotor (Beckman). Saturated $(NH_4)_2SO_4$ was added to the supernatant solution until 20% saturation wa achieved. Precipitates were removed by centrifugation (see above) and were discarded. The supernatant solution was brought to 55% saturation by the addition of saturated $(NH_4)_2SO_4$. The pellet resulting from the subsequent centrifugation was dissolved in TE buffer (10mM Tris-HCl (pH 8.0), 15 mM beta-mercaptoethanol, 1 mM EDTA). The protein solution in TE was then dialyzed against 50 volumes of TE buffer.

After centrifugation (as above) to remove precipitated material, the dialyzed protein solution was applied to a DEAE SEPHAROSE (Pharmacia) column. After washing with TE buffer, proteins were eluted with a 0-300 mM NaCl gradient in TE buffer. Fractions containing an *M. bovis* BCG Hsp60 (actual apparent molecular weight equal to 65 kDa), were identified by SDS-PAGE and Coomassie Blue staining and were pooled. 10 microgram/ml aprotinin, 10 microgram/ml leupeptin, and 1 microgram/ml pepstatin were added to the pool which was then concentrated in an Amicon cell using a YM30 membrane.

The concentrated pool was applied to a S-200 SEPHACRYL (Pharmacia) column equilibrated with S200 buffer (10 mM $Na_2HPO_4$ (pH 6.8), 150 mM NaCl and 15 mM beta-mercaptoethanol). Elution was with the same buffer. Fractions were tested for the presence of mycobacterial Hsp60 as before, and positive fractions containing highly purified protein were pooled and dialyzed overnight against HAP buffer (10 mM $Na_2HPO_4$ (pH 6.8), 15 mM beta-mercaptoethanol).

The dialyzed pool was applied to a hydroxyapatite (Bio-Rad; Bio-Gel HTP Gel) column equilibrated in HAP buffer. The column was washed with 3 column volumes of 1 mN $MgCl_2$ and 15 mM beta-mercaptoethanol and then with 1 mM $Na_2HPO_4$, (pH 6.8) and 15 mM beta-mercaptoethanol. Protein was eluted with a 10-60 mM phosphate gradient. Fractions were tested as before, and positive fractions were pooled, concentrated and exchanged into 0.85% NaCl by means of gel filtration through PD10. The purity of mycobacterial Hsp60 was assessed by SDS-PAGE and Coomassie Blue staining as well as by western blot analysis using antibodies specific for *E. coli* Hsp70 and Hsp60. Preparations were typically more than 90% pure, and contained no more than 0.5% of *E. coli* Hsp60 and 0.1-0.2% E. *coli* Hsp70, respectively.

Hsp preparations can be depyrogenated either by affinity chromatography on DetoxiGel resin, addition of polymyxin B or (least preferably) by extraction with detergents such as Triton® X-114.

Example 2: CTL response to a composition comprising a mixture of an NP peptide and hsp70

a. Preparation of hsp70 and NP peptide Hsp 70, here M. tuberculosis hsp71, was prepared as described in example 1. NP peptide (referred to herein as NP.B; Motal, U.M.A., *et al., Eur. J. Immunol., 25*:1121-1124 (1995) and references therein) with the amino acid sequence VQLASNENMETM (SEQ ID NO: 1) corresponding to residues 363-374 in the complete NP and containing a known CTL epitope (H-2b-restricted) was produced synthetically (0.25 mM scale) on an Applied Biosystems model 431A peptide synthesizer using Fmoc (9-fluorenylmethyloxycarbonyl) as the alpha-amino protective group and HMP (Wang) resin as the solid support. All amino acid and synthesis chemicals were purchased from Applied biosystems.

NP.B was cleaved off the support and side-chain-protecting groups were removed by incubating under continuous agitation NP.B-resin for 3 h in 2ml of a mixture prepared by combining 10 ml trifluoroacetic acid, 0.5 ml water, 0.75 g crystalline phenol, 0.25 ml ethanedithiol and 0.5 ml thioanisole. The cleavage mixture was filtered into 40 ml of ice cold diethyl ether. Insoluble material was collected by centrifugation at 5000 x g for 8 min. Ether was decanted and the pellet washed three times by resuspension in cold diethyl ether followed by centrifugation. After the last wash the pellet was air-dried, taken up in distilled water and lyophilized.

b. Immunization of mice and preparation of effector cells

NP.B peptide was dissolved in a small volume of Dulbecco's PBS (DPBS; 2.7 mM $KH_2PO_4$, 4.3 mM $Na_2HPO_4$, 2.7 mM KCl, 0.137 M NaCl). 1.89, 18.9 or 189 microgram aliquots, respectively, of peptide NP.B were mixed with 100 microgram aliquots of hsp71 in DPBS to obtain compositions with molar ratios of peptide:hsp of 1, 10, or 100, respectively. Groups of four female mice of strain C57BL/6 were either left unimmunized (control) or were injected subcutaneously in the nape of the neck with the three different NP.B-hsp71 mixtures. After seven days, the mice were euthanized by cervical dislocation, and their spleens were removed. Single cell suspensions of pooled spleens were prepared and washed once in 'complete medium', which was RPMI-1640 medium supplemented with 10% fetal bovine serum, 2 mM L-glutamine, 1mM sodium pyruvate, 50 uM 2-mercaptoethanol and 50 ug/ml gentamycin sulfate. Lymphoid cells were restimulated by culturing 25 x $10^6$ viable cells with NP.B peptide at a 0.1 umolar concentration for five days. Cultures were incubated in upright 25 cm2 flasks with 10 ml complete medium at 37°C and 5% CO2. The cultures (effector cells) were then used in the CTL activity assay described below.

c. CTL activity assay

EL4 cells (H-2b) were used as target cells. Cells were incubated for 90 min with 150 uCi $Na_2CrO_4$, and 10 ug NP.B peptide per $10^6$ cells. Following extensive washing to remove excess radiolabel, $10^4$ labeled target-cells were co-cultured with restimulated effector cells at various effector:target cell ratios. After 4-5 hours of incubation, culture plates were centrifuged for 5 min at 200 x g, and 100 ul aliquots of supernatant solutions containing radiolabel released from cells were collected into Beckman Ready Caps. Radioactivity was measured by liquid scintillation counting. To determine spontaneously released and total releasable radioactivity, supernatant solutions from cultures containing target cells only or from target cells lysed by the addition of Triton® X-100 were collected, and radioactivity determined as before. Results were expressed as % specific lysis, calculated based on the following formula:

$$\text{Percent Specific lysis} = 100 \times (\text{cpmtest} - \text{cpmspont})/(\text{cpmtotal} - \text{cpmspont}),$$

wherein cpmtest is the radioactivity released from a particular co-culture, cpmspont is the spontaneously released radioactivity of a target cell culture and cpmtotal is the radioactivity released by Triton X-100 lysis of target cells. CTL assays were performed in triplicate, and averaged value were provided.

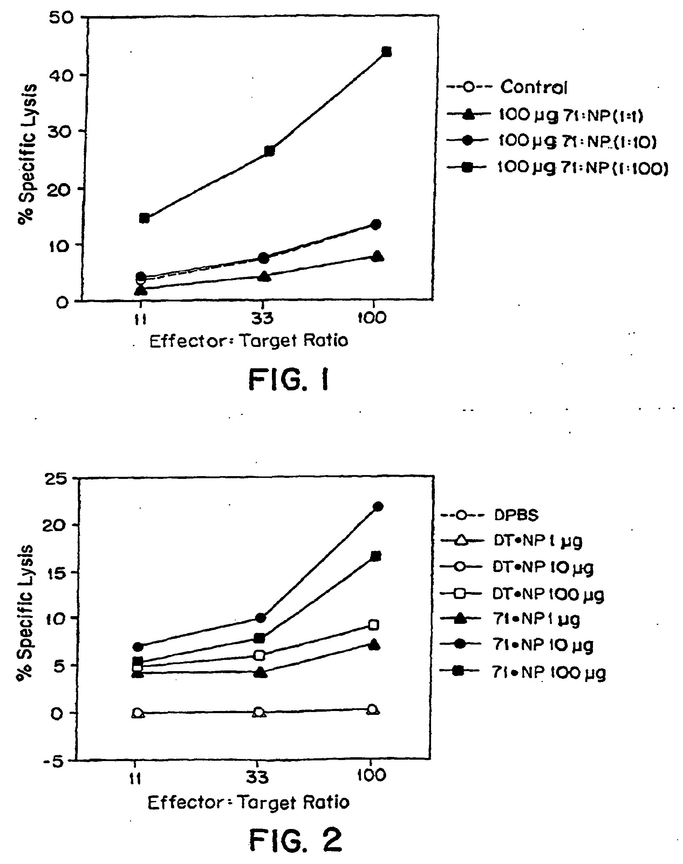

Results of the experiment are shown in Fig. 1. The control reaction, i.e., assay of chromium release of a co-culture of target cells and effector cells prepared from unimmunized mice, provides a background value for lysis of about 10% at an effector:target cell ratio of 100. No enhancement of CTL activity over background was observed with effector cells from mice immunized with 1:1 or 10:1 NP.B-hsp71 mixtures. Greatly enhanced lysis was found with effector cells from mice immunized with a 100:1 mixture of NP.B peptide and hsp71, demonstrating that co-immunization with a peptide such as NP.B and an hsp such as hsp71 can drastically stimulate CTL activity against cells displaying the peptide. Note that, as is well known in the field, immunization with NP.B peptide in DPBS alone does not stimulate CTL activity.

Example 3: CTL response to a composition comprising a chemical conjugate of an NP peptide and hsp70

a. Preparation of hsp70 and NP peptide

M. tuberculosis hsp71 was prepared as described in Example 1. NP.B peptide was synthesized as discussed in Example 2, except that the peptide contained an extra amino-terminal cysteine residue and, thus, had the amino acid sequence CVQIASNENMETM (SEQ ID NO: 2).

b. Chemical conjugation of Np.B peptide to hsp70 and diphtheria toxoid

Conjugations were carried out with both hsp70 and, to provide a standard for comparisons of efficacies of specific stimulation of CTL activity, commonly used carrier protein diphtheria toxoid (abbreviated DT; DT was obtained from a commercial source).

b.1. Activation of M. tuberculosis hsp71 and DT carrier proteins

Nine mg of hsp71 were dissolved in 4.5 ml of 0.1 M sodium borate buffer, pH 8.0. Sulfo-MBS (m-maleimidobenzoyl-N-hydroxysulfosuccinimide ester) (2.3 mg in 100 ul dimethyl sulfoxamine) was added to the protein, and the reaction mixture was incubated for 1 hour at room temperature. The pH was then adjusted to 6.0, and the reaction mixture dialyzed overnight at 4°C against 1 liter of 20 mM sodium phosphate and 150 mM NaCl, pH 5.6. DT was similarly treated.

b.2. Preparation of NP.B peptide for conjugation

For each conjugation reaction, 3 mg of peptide was dissolved in 100 ul of 0.1 M beta-mercaptoethanol. After 1 hour of incubation to allow reduction of the peptide, reducing agent was removed by drying the reaction mixture in a SpeedVac centrifuge. Peptide was redissolved in 0.5 ml distilled water to which 5 ul aliquots of 1 N NaOH were added until the peptide was fully dissolved. For conjugation experiments with DT, 6 mg of peptide were reduced and then redissolved in 1 ml of water.

b.3. Conjugate formation

The pH of the activated carrier protein solutions was adjusted to 6.8 using 0.1 N NaOH. Solution containing 3 mg of activated carrier protein was reacted with 0.5 ml of reduced peptide solution (or 1 ml of reduced peptide solution for the preparation of conjugates with DT) for 3 hours at room temperature with continuous mixing. To remove unreacted peptide, the resulting conjugate-containing solution was dialyzed overnight at 4°C against 1 liter of 20 mM sodium phosphate and 150 mM NaCl, pH7. Protein concentration was determined by BCA assay. The efficiency of conjugation achieved by this procedure had been determined in prior pilot experiments using radiolabeled NP.B peptide. The peptide: protein ratio was found to be 17.5 for NP.B-hsp71 conjugate (71.NP) and 10.1 for NP.B-DT (DT.NP).

c. Immunization of mice and preparation of effector cells

Immunizations with 1-100 ug of 71.NP and DT.NP conjugates and preparation of effector cells were performed as described in Example 2.

d. CTL activity assay

Assays were performed as described in Example 2.

Results

Results obtained are displayed in Fig. 2. CTL activity assays with effector cells from mice injected with DPBS or with 1 or 10 ug of DT.NP conjugate gave negative results (lowest line Fig.2). Only effector cells injected with 100 ug of DT.NP produced measurable (between 5 and 10% lysis at an effector: target cell ratio of 100) CTL activity that was comparable to that of effector cells from mice immunized with 1 ug of 71.NP. Assays with effector cells from mice immunized with 10 or 100 ug of 71.NP conjugate showed substantially greater CTL activity i.e., between 15 and 25% target cell lysis at an effector:target cell ratio of 100. This experiment demonstrates on the example of the NP.B peptide and hsp71 that immunization with a peptide-hsp conjugate stimulates specific CTL activity directed against cells displaying the peptide.

Example 4: CTL response to a composition comprising an hsp-NP fusion protein

a. Preparation of hsp-NP fusion proteins

a.1. Preparation of expression plasmids encoding fusion proteins containing NP CTL epitopes at the carboxy terminus of mycobacterial hsp65

Plasmids expressing as part of hsp65 fusion proteins influenza virus NP sequences including the H-2b CTL epitope NP.B (see above) or the H-2$^d$ CTL epitope NP.D (residues 147-155 of NP; Levi, R. and Arnon, R., *Vaccines, 14*:85-92 (1996) and references therein) were constructed.

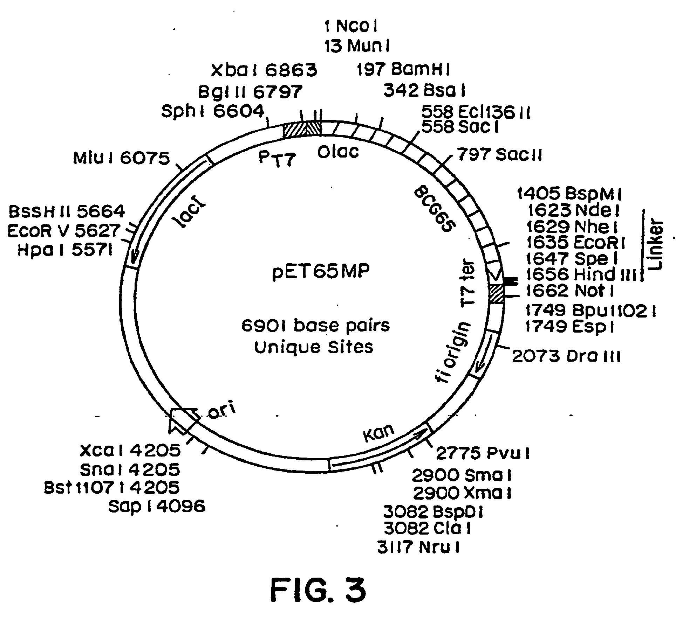

An expression vector, pET65mp, derived from a pET system plasmid (Novagen) and containing a complete M. *bovis* BCG hsp65 gene and useful restriction sites for insertion of additional coding sequences at the carboxy terminus of the hsp65 gene was previously constructed. A schematic representation of this vector is provided in Fig. 3.

Construct pNP/cA containing the open reading frame of NP of influenza virus strain A/PR/8/34 under the control of the cytomegalovirus promoter provided by plasmid pcDNA1 (Invitrogen) was obtained from Dr. Peter Palese (Dept. Of Microbiology, Mount Sinai School of Medicine, New York, NY).

Two primer pairs for amplification of fragments containing the NP.B and NP.D epitopes were synthesized on an automated oligonucleotide synthesizer and were purified using routine procedures. Forward primers contained, in addition to appropriate sequences complementary to NP sequences, an EcoRI restriction site, and reverse primers a SpeI restriction site. The forward primer for the NP.D fragment had the sequence 5' AAAGAAGAATTCAGGCGAATC (SEQ ID NO: 3), and the reverse primer the sequence 5' GTTCCGATCACTAGTCCCACG (SEQ ID NO: 4). This pair was designed to amplify a fragment containing NP residues 117-200. The forward primer for the NP.B fragment had the sequence 5' CTGCTTGAATTCAGCCAAGTG (SEQ ID NO: 5), and the reverse primer the sequence 5' CTGTTGACTAGTGTTTCCTCC (SEQ ID NO: 6). The latter pair was designed to produce a fragment containing NP residues 310-395.

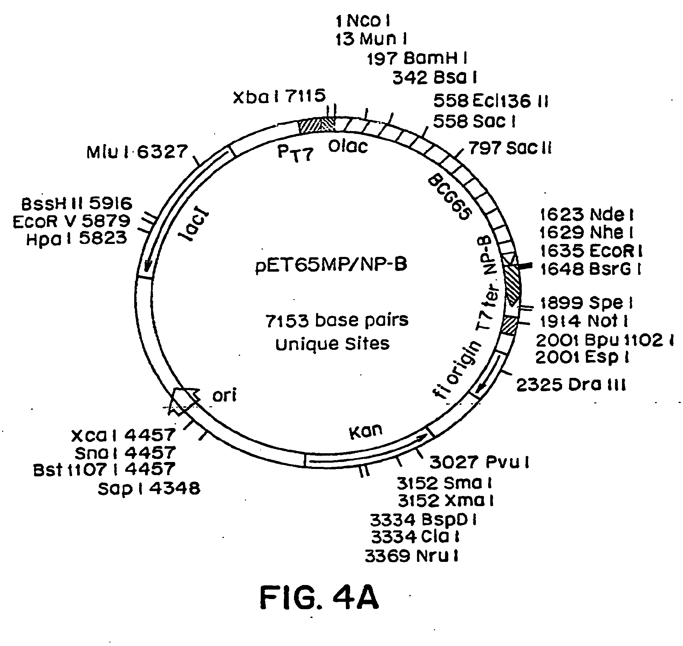

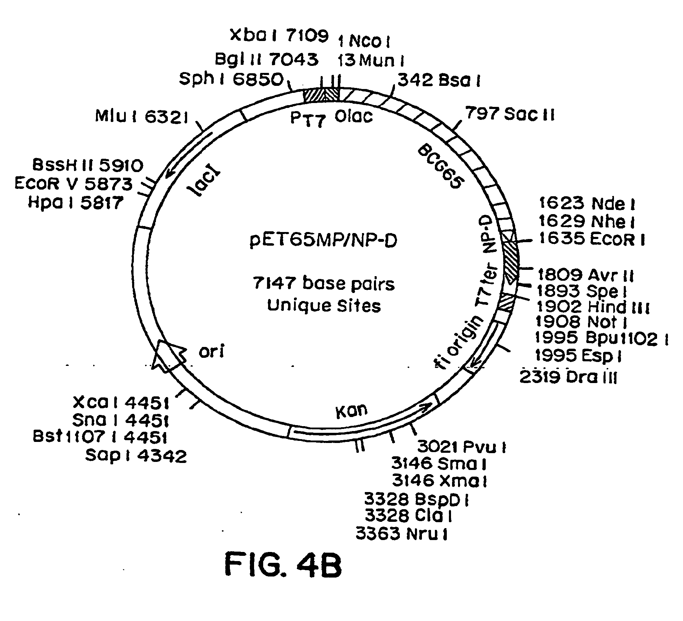

Polymerase chain reactions (PCR) were carried out using the above primer pairs and pNP/cA as the DNA template. PCR fragments were double-digested with restriction endonucleases EcoRI and SpeI and ligated to EcoRI/ SpeI-cut pET65mp using routine subcloning procedures (Maniatis *et al.*, *Molecular Cloning, A Laboratory Manual,* Cold Spring Harbor Lab., Cold Spring Harbor, NY (1989)). Transformation-competent cells of *E. coli* strain DH5alpha were transformed with the ligation mixtures and plated out on agar containing 100 ug/ml ampicillin. Colonies of transformed cells were isolated, and plasmid DNA prepared and analyzed for the presence of the correct hsp65-NP.B or D fusion gene sequence by restriction mapping and nucleotide sequencing. Correct constructs encoding hsp65-NP.B (pET65mp/B) and hsp65-NP.D (pET65mp/D) fusion proteins were identified and were used in subsequent manipulations aimed at expression of fusion proteins in bacteria and their purification. See Figs. 4A-4B for schematic representations of the fusion protein gene constructs, pET65MP/NP-B and pET65MP/NP-D, respectively.

a.2. Expression and purification of hsp65-NP fusion proteins

Fusion protein constructs were transformed into *E. coli* strain BL21 (DE3; Novagen), and fusion proteins were expressed in 6 liter cultures of the latter strain, using a protocol closely similar to the supplier's suggested protocol. Cells were harvested by centrifugation, suspended in 10 mM Tris-HCl, 2 mM EDTA, and 5 mM beta-mercaptoethanol, pH 7.5 and lysed by sonication. After removing insoluble material by centrifugation, ammonium sulfate was added to 20% saturation, and precipitating proteins were collected by centrifugation. The presence of fusion protein in the ammonium sulfate pellet was verified by SDS-PAGE followed by Coomassie blue staining. The same assay was used to monitor all subsequent purification steps. Protein was redissolved in 30 mM Tris-HCl, 2mM EDTA and 5 mM beta-mercaptoethanol, pH 7.5, and the solution was exhaustively dialyzed against the same buffer before being applied to a DEAE Sepharose (fast flow, Pharmacia Biotech) column equilibrated in the same buffer. The flow-through fraction (unbound protein) was collected which typically contained about 90 mg of protein. To further purify hsp65-NP.B fusion protein, 60 mg of the latter fraction was dialyzed against 10 mM sodium phosphate, pH 6.8 and then applied to a hydroxyapatite (BIORAD) column equilibrated in the same buffer. Elution was performed using a 0-600 mM potassium phosphate gradient. This procedure resulted in the recovery of only about 5 mg protein. The column was then further eluted with 4 M guanidinium hydrochloride which removed another 15 mg of protein. The fractions were dialyzed against DPBS and concentrated using an Amicon ultrafiltration device, before being applied to a Detoxiel column for flow-through depyrogenation. To further purify hsp65-NP.D fusion protein, DEAE Sepharose flow-through fraction was dialyzed against 30 mM sodium acetate, 2mM EDTA, and 5mM beta-mercaptoethanol, pH 5.8-7.5 and then applied to an SP Sepharose (fast flow, Pharmacia Biotech) column equilibrated in the same buffer. Elution was with a 0-600 mM NaCl gradient. Eluted hsp65-NP.D fusion protein was processed as described for hsp65-NP.B fusion protein. Fusion proteins purified by these procedures were more than 90% pure as estimated from stained SDS-PAGE gels and were substantially pyrogen-free.

b. Immunization of mice and preparation of effector cells

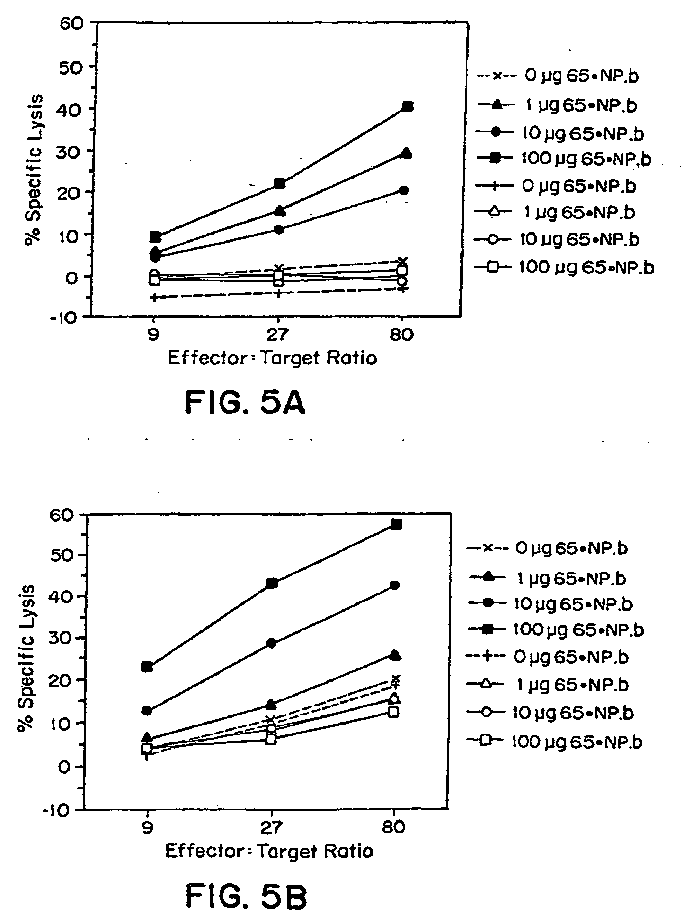

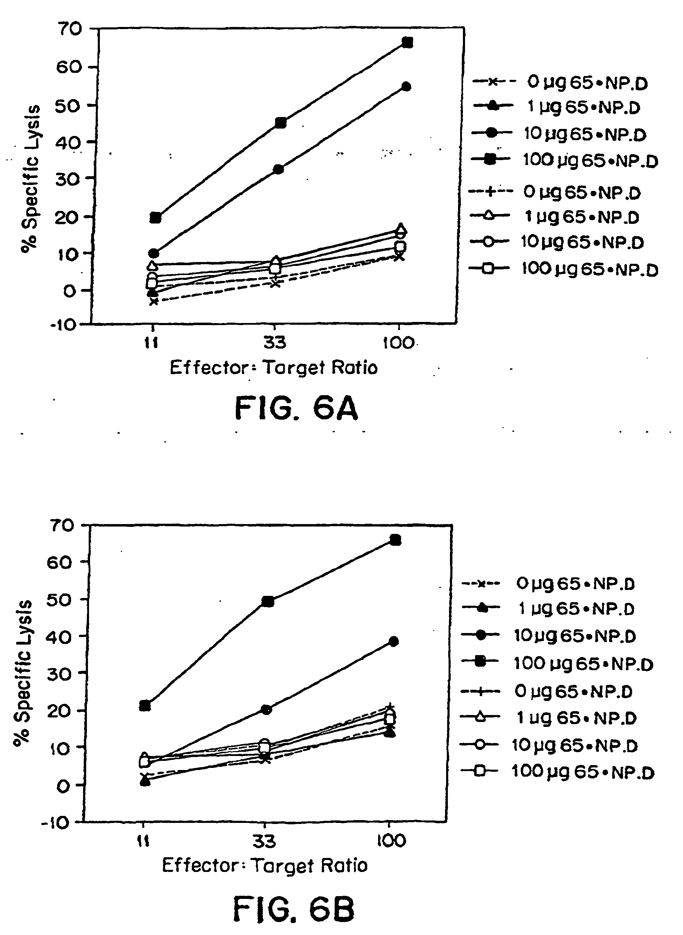

Immunizations with DPBS (referred to in Figs. 5 & 6 as 0 ug 65-NP) or 1-100 ug of hsp65-NP.B or hsp65-NP.D fusion proteins and preparation of effector cells were performed essentially as described in Example 2, except that C57BL/6 mice were used in experiments with hsp65-NP.B, and BALB/c mice in experiments with hsp65-NP.D. *In vitro* restimulation was carried out over a period of seven days, either in the absence or in the presence of 3U/ml of recombinant human IL2 (to generally stimulate T cell proliferation).

c. CTL assays

Assays were performed essentially as described in Example 2, except that EL4 (H-2b) target cells were used in experiments with hsp65-NP.B fusion protein and P815 (H-2$^d$) target cells in experiments with hsp65-NP.D fusion protein. To provide an additional control for the specificity of the CTL response, target cells were either pulsed with the appropriate NP peptide (closed symbols in Figs. 5A-5B & 6A-6B), were pulsed with the irrelevant residue 49-to-57-peptide derived from the sequence of the HPV16E7 protein (open symbols in Figs. 5A-5B) or were not pulsed (open symbols in Figs. 6A-6B).

The results of experiments with hsp65-NP.B fusion protein (labeled 65-P.b) are shown in Figs. 5A-5B. Fig. 5A refers to an experiment in which effector cells were restimulated in the absence, and Fig. 5B refers to an experiment in which effector cells were restimulated in the presence of IL2. As is evident from Fig. 5A, immunization with hsp65-NP.B fusion protein results in a dramatic stimulation of specific CTL activity directed against target cells displaying the NP.B peptide, ranging from about 20 to 40% lysis of target cells at an effector:target cell ratio of 100. Essentially no specific lysis was observed with effector cells from DPBS- "immmunized" animals. Also, no significant lysis of E7-peptide-pulsed cells was evident. In the experiment with effector cells restimulated in the presence of IL2, even higher levels of target cell lysis were observed with effector cells from hsp65-NP.B fusion protein-immunized mice. Levels ranged from about 25 to 60% at an effector:target cell ratio of 100, depending on the fusion protein dose. These values greatly exceed the 10-15% lysis observed with effector cells from DPBS-injected mice. Again, no significant (greater than that observed with effector cells from DPBS- "immunized" mice), specific lysis of E7 peptide-pulsed target cells was observed.

Results of experiments with hsp65-NP.D fusion protein (labeled 65-NP.D) are shown in Figs. 6A-6B. Generally, these results are similar to those obtained in experiments with the hsp65-NP.B fusion protein. Note that, unlike in the previous experiment with hsp65-NP.B, a clear dependence on the dose of hsp65-NP.D peptide used in immunization was observed in this experiment. Together, these experiments, using hsp65-NP fusion proteins as examples, demonstrate that immunization with an hsp-foreign peptide/polypeptide fusion protein results in a drastic stimulation of CTL activity directed against appropriate target cells displaying epitopes contained in the foreign peptide/polypeptide fusion partner.

Example 5: CTL responses to an hsp-P1A fusion protein

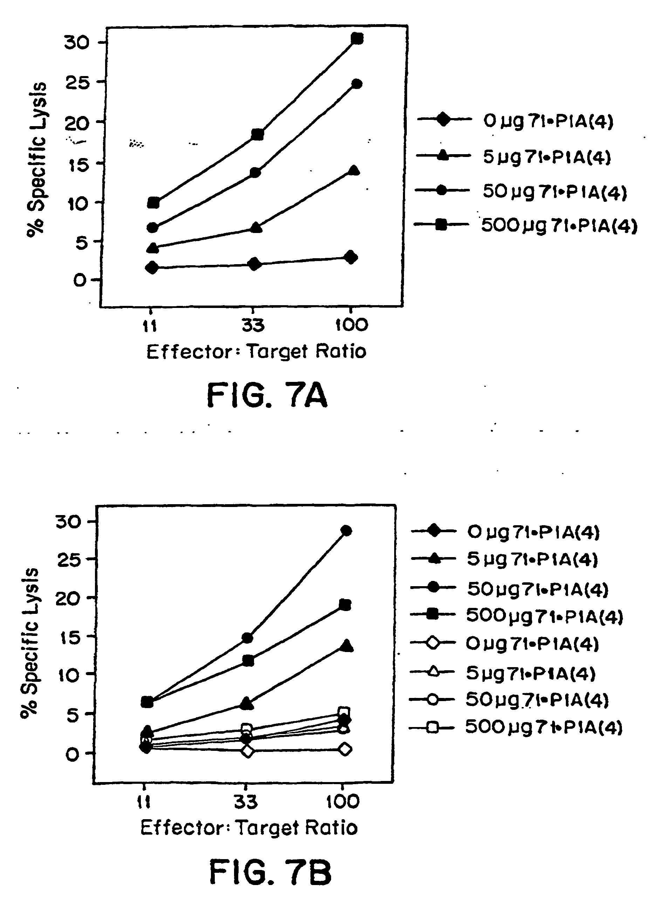

Using procedures similar to those used in the preceding example, a plasmid was constructed that permitted expression in *E. coli* of a fusion gene containing the complete coding sequence of M. tuberculosis stress protein hsp71 and, added to the carboxy end of the hsp71 sequence, four tandemly arranged copies of a synthetic sequence encoding the minimal CTL epitope of tumor-associated antigen P1A (LPYLGWLVP (SEQ ID NO: 7); this seuqence is referred to as P1A in this example). Hsp71-P1A fusion protein (referred to as 71-P1A(4) in Figures 7A, 7B, 8A, 8B and 9) was expressed and purified using standard biochemical methods similar to those used in the preceding example.

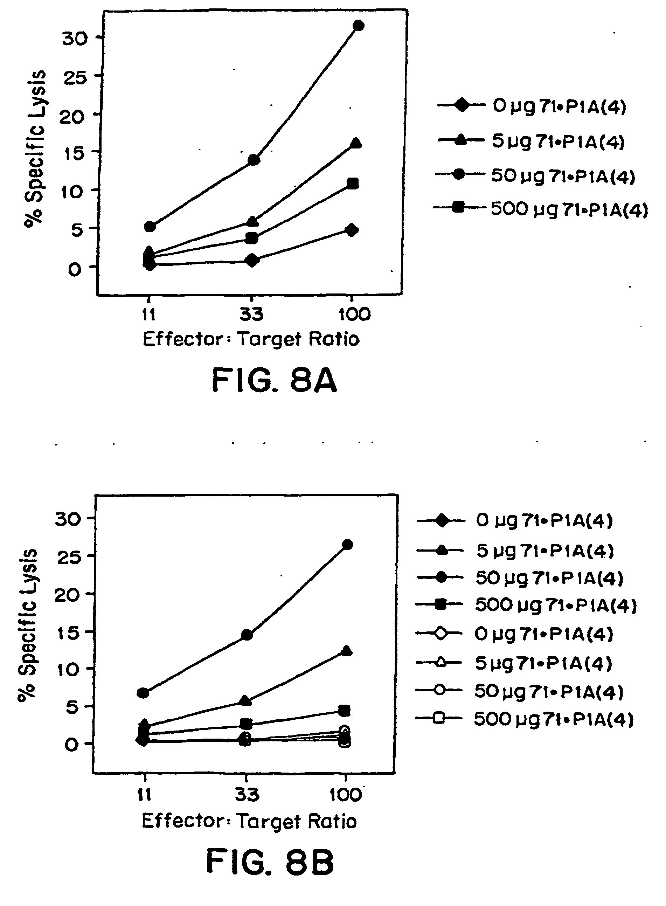

BALB/c or DBA/2 (H-2$^d$) mice were anesthetized by intraperitoneal injection of ketamine hydrochloride. The mice were then immunized subcutaneously in the nape of the neck with 0, 5, 50 or 500 $\mu$g of hsp71-P1A fusion protein. The immunogen was administered in DPBS without adjuvant. One week later, single cell suspensions form four pooled spleens per group were prepared and restimulated in vitro for 7 days with synthetic peptide CKKKLPYLGWLVP (SEQ ID NO: 8) (1 $\mu$M). Note that the CKKK residues were added to enhance the aqueous solubility of the P1A nonamer. Restimulated effector cells were then cultured for 4-5 hours with $^{51}$Cr-labelled target cells. Targets were cells of the P1A antigen-expressing clone P1 (H-2$^d$) of the P815 mastocytoma, or, alternatively, L1210 cells (H-2$^d$) pulsed with (CKKK) P1A or, as control targets, unpulsed L1210 cells at effector:target ratios of 100, 33 or 11:1. Specific lysis of target cells was determined as described in Example 2. The results of these experiments are represented in Figures 7A-7B (BALB/c mice) and 8A-8B (DBA/2 mice). Background lysis in these experiments was less that 5%, as indicated by the lytic activity observed against irrelevant target cells (unpulsed L1210 cells). Restimulated cells from unimmunized mice (0 $\mu$g) exhibited no lytic activity against either P1 target cells (Figures 7A, 8A) or CKKK (P1A)-pulsed L1210 cells. (Figures 7B, 8B). Cells from mice immunized with as little as 5 $\mu$g of hsp71-P1A fusion protein exhibited measurable lytic activity, with the maximal response seen in cells from mice immunized with 50-500 $\mu$g.

Example 6: Tumor challenge of mice immunized with hsp71-P1A fusion protein

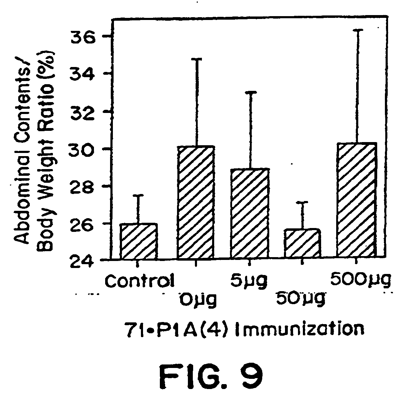

Mice were immunized as in the preceding example, except that three injections were given at intervals of two weeks. Two weeks after the final injection, the mice were challenged by intraperitoneal injection of 1000 viable P1 tumour cells. After 26 days, the mice were euthanized, weighed, and the entire mass of abdominal contents was dissected and weighed. The results of this experiment are shown in Figure 9. It was observed that in mice given three 50 $\mu$g immunizations with hsp71-P1A fusion-protein, the mass of the abdominal contents, as expressed as a percentage of the total body weight, was significantly less than that found in unimmunized mice (0 $\mu$g, P<0.03), and similar to that observed in mice which were not injected with tumor cells (control).

Together, the experiments in Examples 5 and 6 demonstrate that immunization with hsp-tumor-associated antigen, using hsp71-P1A as the example, results in substantial stimulation of CTL activity directed against cells displaying irrelevant MHC class I-restricted epitopes. Further, such immunization leads to the expression of a relevant effector function, namely immunity against challenge with a tumor expressing the antigen immunized against.

**Claims**

1. A fusion protein comprising an antigen of an influenza virus and a stress protein, or a fragment thereof provided such fragment includes the conformational epitopes involved with enhancing the immune response to the said antigen thereof wherein the fusion protein induces an immune response against the antigen in a mammal to whom the fusion protein is administered.

2. The fusion protein of claim 1, wherein the antigen of the influenza virus is hemagglutinin, nucleoprotein, neuraminidase, M1, M2, PB1, PB2, or PA.

3. The fusion protein of claim 1, wherein the fusion protein is encoded by plasmid pET65MP/NP-B as defined in Fig. 4A or plasmid pET65MP/NP-D as defined in Fig. 4B.

4. The fusion protein of claim 1, wherein the stress protein is an Hsp100-200, an Hsp100, an Hsp90, Lon, an Hsp70, an Hsp60, TF55, an Hsp40, an FKBP, a cyclophillin, an Hsp20-30, C1pP, GrpE, Hsp10, ubiquitin, calnexin, or a protein disulfide isomerase.

5. The fusion protein of claim 1, wherein the bacterial stress protein is a mycobacterial stress protein.

6. The fusion protein of claim 5, wherein the mycobacterial stress protein is hsp65.

7. The fusion protein of claim 5, wherein the mycobacterial stress protein is hsp71.

8. The fusion protein of claim 1, wherein the stress protein is a heat shock protein.

9. The fusion protein of claim 1, wherein the antigen comprises at least one B cell epitope.

10. The fusion protein of claim 1, wherein the antigen comprises at least one T cell epitope.

11. The fusion protein of claim 10, wherein the antigen comprises at least one CTL cell epitope.

12. The fusion protein of claim 1, wherein the immune response is a cell mediated immune response.

13. The fusion protein of claim 12, wherein the cell mediated immune response is a cell mediated cytolytic immune response.

14. The fusion protein of claim 1, wherein the immune response is a T cell helper or a B cell immune response.

15. A composition comprising the fusion protein of any one of the preceding claims and a pharmaceutically acceptable excipient, carrier, diluent, or vehicle.

16. A polynucleotide encoding the fusion protein of any one of claims 1 to 14.

17. The fusion protein of any one of claims 1 to 14 for use in inducing an immune response in a mammal.

18. The polynucleotide of claim 16 for use in inducing an immune response in a mammal.

19. In-vitro use of a polynucleotide of claim 16 for producing a fusion protein according to any one of claims 1 to 14.

**Patentansprüche**

1. Fusionsprotein, umfassend ein Antigen eines Influenzavirus und ein Stressprotein oder ein Fragment davon, mit der Maßgabe, dass solch ein Fragment die Konformations-Epitope enthält, welche in die Verstärkung der Immunantwort auf das Antigen involviert sind, wobei das Fusionsprotein eine Immunantwort gegen das Antigen in einem Säuger auslöst, welchem das Fusionsprotein verabreicht wurde.

2. Fusionsprotein nach Anspruch 1, wobei das Antigen des Influenzavirus Hämagglutinin, Nucleoprotein, Neuraminidase, M1, M2, PB1, PB2 oder PA ist.

3. Fusionsprotein nach Anspruch 1, wobei das Fusionsprotein von Plasmid pET65MP/NP-B, wie in Figur 4A gezeigt, oder Plasmid pET65MP/NP-D, wie in Figur 4B gezeigt, codiert wird.

4. Fusionsprotein nach Anspruch 1, wobei das Stressprotein ein Hsp100-200, ein Hsp100, ein Hsp90, Lon, ein Hsp70, ein Hsp60, TF55, ein Hsp40, ein FKBP, ein Cyclophillin, ein Hsp20-30, C1pP, GrpE, Hsp10, Ubiquitin, Calnexin oder eine Proteindisulfidisomerase ist.

5. Fusionsprotein nach Anspruch 1, wobei das bakterielle Stressprotein ein mycobakterielles Stressprotein ist.

6. Fusionsprotein nach Anspruch 5, wobei das mycobakterielle Stressprotein Hsp65 ist.

7. Fusionsprotein nach Anspruch 5, wobei das mycobakterielle Stressprotein Hsp71 ist.

8. Fusionsprotein nach Anspruch 1, wobei das Stressprotein ein Hitzeschockprotein ist.

9. Fusionsprotein nach Anspruch 1, wobei das Antigen mindestens ein B-Zell-Epitop umfasst.

10. Fusionsprotein nach Anspruch 1, wobei das Antigen mindestens ein T-Zell-Epitop umfasst.

11. Fusionsprotein nach Anspruch 10, wobei das Antigen mindestens ein CTL-Zell-Epitop umfasst.

12. Fusionsprotein nach Anspruch 1, wobei die Immunantwort eine zellvermittelte Immunantwort ist.

13. Fusionsprotein nach Anspruch 12, wobei die zellvermittelte Immunantwort eine zellvermittelte cytolytische Immunantwort ist.

14. Fusionsprotein nach Anspruch 1, wobei die Immunantwort eine T-Helferzell- oder eine B-Zell-Immunantwort ist.

15. Zusammensetzung, umfassend das Fusionsprotein nach einem der vorausgehenden Ansprüche und einen pharmazeutisch verträglichen Excipienten oder Träger oder ein pharmazeutisch verträgliches Verdünnungsmittel oder Vehikel.

16. Polynucleotid, welches das Fusionsprotein nach einem der Ansprüche 1 bis 14 codiert.

17. Fusionsprotein nach einem der Ansprüche 1 bis 14 zur Verwendung bei der Auslösung einer Immunantwort in einem Säuger.

18. Polynucleotid nach Anspruch 16 zur Verwendung bei der Auslösung einer Immunantwort in einem Säuger.

19. In-vitro-Verwendung des Polynucleotids nach Anspruch 16 zur Herstellung eines Fusionsproteins nach einem der Ansprüche 1 bis 14.

**Revendications**

1. Protéine de fusion comprenant un antigène d'un virus influenza et une protéine de stress ou un fragment de celle-ci, ledit fragment incluant les épitopes conformationnels impliqués dans l'augmentation de la réponse immune contre ledit antigène, ladite protéine de fusion induisant une réponse immune contre l'antigène chez un mammifère auquel la protéine de fusion est administrée.

2. Protéine de fusion selon la revendication 1, dans laquelle l'antigène du virus influenza est l'hémagglutinine, la nucléoprotéine, la neuraminidase, M1, M2, BP1, BP2 ou PA.

3. Protéine de fusion selon la revendication 1, dans laquelle la protéine de fusion est codée par le plasmide pET65MP/NP-B tel que défini dans la Fig. 4A ou le plasmide pET65MP/NP-D tel que défini dans la Fig. 4B.

4. Protéine de fusion selon la revendication 1, dans laquelle la protéine de stress est une Hsp100-200, une Hsp100, une Hsp90, Lon, une Hsp70, une Hsp60, TF55, une Hsp40, une FKBP, une cyclophilline, une Hsp20-30, C1pB, GrpE, Hsp10, ubiquitine, calnexine, ou une protéine disulfure isomérase.

5. Protéine de fusion selon la revendication 1, dans laquelle la protéine de stress bactérienne est une protéine de stress mycobactérienne.

**6.** Protéine de fusion selon la revendication 5, dans laquelle la protéine de stress mycobactérienne est hsp65.

**7.** Protéine de fusion selon la revendication 5, dans laquelle la protéine de stress mycobactérienne est hsp71.

**8.** Protéine de fusion selon la revendication 1, dans laquelle la protéine de stress est une protéine de choc thermique.

**9.** Protéine de fusion selon la revendication 1, dans laquelle l'antigène comprend au moins un épitope de cellule B.

**10.** Protéine de fusion selon la revendication 1, dans laquelle l'antigène comprend au moins un épitope de cellule T.

**11.** Protéine de fusion selon la revendication 10, dans laquelle l'antigène comprend au moins un épitope de cellule CTL.

**12.** Protéine de fusion selon la revendication 1, dans laquelle la réponse immune est une réponse immune à médiation cellulaire.

**13.** Protéine de fusion selon la revendication 12, dans laquelle la réponse immune à médiation cellulaire est une réponse immune cytolytique à médiation cellulaire.

**14.** Protéine de fusion selon la revendication 1, dans laquelle la réponse immune est une réponse immune à cellule T auxiliaire ou à cellule B.

**15.** Composition comprenant la protéine de fusion selon l'une quelconque des revendications précédentes et un excipient, support, diluant ou véhicule acceptable sur le plan pharmaceutique.

**16.** Polynucléotide codant la protéine de fusion selon l'une quelconque des revendications 1 à 14.

**17.** Protéine de fusion selon l'une quelconque des revendications 1 à 14, pour une utilisation pour induire une réponse immune chez un mammifère.

**18.** Polynucléotide selon la revendication 16, pour une utilisation pour induire une réponse immune chez un mammifère.

**19.** Utilisation in vitro d'un polynucléotide selon la revendication 16 pour produire une protéine de fusion selon l'une quelconque des revendications 1 à 14.

FIG. 1

FIG. 2

1 NCO I
13 Mun I

Xba I 6863      197 BamH I

Bgl II 6797      342 Bsa I

Sph I 6604      558 Ecl136 II
558 Sac I

Miu I 6075      P_T7   Olac      797 Sac II

BCG65

Mlu I 6075

BssH II 5664      1405 BspM I
EcoR V 5627      1623 Nde I
Hpa I 5571      1629 Nhe I
1635 EcoR I
1647 Spe I
pET 65 MP      1656 Hind III
1662 Not I
6901 base pairs      1749 Bpu1102 I
Unique Sites      1749 Esp I

2073 Dra III

ori

Xca I 4205      Kan
Sna I 4205
Bst1107 I 4205      2775 Pvu I
Sap I 4096      2900 Sma I
2900 Xma I
3082 BspD I
3082 Cla I
3117 Nru I

# FIG. 3

1 Nco I
13 Mun I
197 BamH I
342 Bsa I
558 Ecl136 II
558 Sac I
797 Sac II

Xba I 7115

Mlu I·6327

P_T7    Olac

BssH II 5916
EcoR V 5879
Hpa I 5823

lacI

BCG65

fl origin T7 ter. NP-B

pET65MP/NP-B

7153 base pairs
Unique Sites

1623 Nde I
1629 Nhe I
1635 EcoR I
1648 BsrG I

1899 Spe I
1914 Not I
2001 Bpu 1102 I
2001 Esp I

2325 Dra III

ori

Kan

Xca I 4457
Sna I 4457
Bst 1107 I 4457
Sap I 4348

3027 Pvu I
3152 Sma I
3152 Xma I
3334 BspD I
3334 Cla I
3369 Nru I

# FIG. 4A

Xba I 7109   1 NCO I
Bgl II 7043   13 Mun I
Sph I 6850   342 Bsa I
Mlu I 6321   PT7   Olac   797 Sac II
BssH II 5910
EcoR V 5873
Hpa I 5817

lacI

pET65MP/NP-D

7147 base pairs
Unique Sites

BCG65

1623 Nde I
1629 Nhe I
1635 EcoR I

1809 Avr II
1893 Spe I
1902 Hind III
1908 Not I
1995 Bpu1102 I
1995 Esp I
2319 Dra III

ori

Xca I 4451
Sna I 4451
Bst1107 I 4451
Sap I 4342

Kan

3021 Pvu I
3146 Sma I
3146 Xma I
3328 BspD I
3328 Cla I
3363 Nru I

T7ter NP-D

fi origin

FIG. 4B

FIG. 5A

FIG. 5B

FIG. 6A

FIG. 6B

FIG. 7A

FIG. 7B

FIG. 8A

FIG. 8B

FIG. 9